(19) Europäisches Patentamt
European Patent Office
Office européen des brevets

(11) EP 1 605 060 A1

(12) **EUROPEAN PATENT APPLICATION**

(43) Date of publication:
**14.12.2005 Bulletin 2005/50**

(51) Int Cl.[7]: **C12Q 1/68**

(21) Application number: **04102641.0**

(22) Date of filing: **10.06.2004**

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(71) Applicant: **Biodynamics S.R.L.**
**C1085ABQ Buenos Aires (AR)**

(72) Inventor: **Mautner, Martin Eduardo,**
**Biodynamics S.R.L.**
**C1085ABQ Buenos Aires (AR)**

(74) Representative: **Ponti Sales, Adelaida et al**
**Oficina Ponti**
**C. Consell de Cent, 322**
**08007 Barcelona (ES)**

(54) **Methods and reaction mixture reagent for increasing the specificity and fidelity of polymerase reactions**

(57) A method for reducing the efficiency of primer extension by polymerase enzymes when the 3' end of a primer or growing nucleic acid chain does not hybridize perfectly with the target, for increasing the selectivity of single nucleotide mutation or gene analyses, for suppressing false positive results and for enhancing the fidelity of the amplification of nucleic acid fragments by avoiding the incorporation of mispairs, the method comprising the steps of: (a) obtaining a nucleic acid sample; (b) hybridizing said nucleic acid sample to a primer; (c) subjecting said nucleic acid sample hybridized to a extension reaction by extending a primer with a polymerizing enzyme; and (d) detecting the presence of extension products; wherein the reaction extension mixture medium contains an intercalating agent such as ethidium bromide, dihydroethidium, ethidium homodimer-1, ethidium homodimer-2, acridine, propidium iodide, YOYO®-1 or TOTO®-1. When the intercalating agent is ethidium bromide the concentration is about 1 to 7µg/ml. A reaction extension mixture reagent is also provided, the reagent comprising a polymerizing enzyme, dNTPs, a buffer, an intercalating agent such as ethidium bromide. The reaction extension mixture reagent may be a PCR reaction mixture or any medium which an extension reaction of nucleic acid was made.

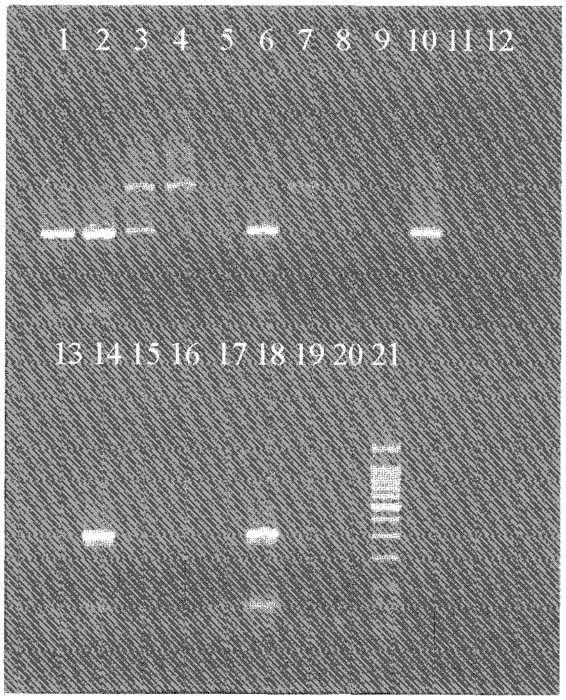


FIG. 1

EP 1 605 060 A1

SEQUENCE LISTING

<110> Martin Mautner
Mautner, Martin

<120> Methods and reaction mixture reagent for increasing the fidelity and specificity of polymerase extension reaction

<130> Mautner, Martin

<160> 27

<170> PatentIn version 3.2

<210> 1
<211> 20
<212> DNA
<213> Artificial

<220>
<223> Human mitochondrial D-loop (16,031-16,330, carrying cytosine nucleotide (C) at position 16311) upstream primer

<400> 1
atggggaagc agatttgggt 20

<210> 2

<211> 20

<212> DNA

<213> Artificial

<220>

<223> Human mtDNA (219 bp fragment of region II, 16513-162) upstream primer

<400> 2

tcagggtcat aaagcctaaa 20

<210> 3

<211> 20

<212> DNA

<213> Artificial

<220>

<223> Human mitochondrial D-loop (16,031-16,330, carrying adenosine nucleotide (A) at position 16311) downstream primer

<400> 3

acggtaaatg gctttatgta 20

<210> 4

<211> 20

<212> DNA

<213> Artificial

<220>

<223> Human mitochondrial D-loop (16,031-16,330, carrying guanosine nucleotide (G) at position 16311) downstream primer

<400> 4

acggtaaatg gctttatgtg 20

<210> 5

<211> 20

<212> DNA

<213> Artificial

<220>

<223> Human mitochondrial D-loop (16,031-16,330, carrying thymidine nucleotide (T) at position 16311) downstream primer

<400> 5

acggtaaatg gctttatgtt 20

<210> 6

<211> 20

<212> DNA

<213> Artificial

<220>

<223> Human mitochondrial D-loop (16,031-16,330, carrying cytosine nucleotide (C) at position 16311) downstream primer

<400> 6

acggtaaatg gctttatgtc 20

<210> 7

<211> 20

<212> DNA

<213> Artificial

<220>

<223> Human mtDNA (244 bp fragment of region I, 16,031-16,275, carrying adenosine nucleotide (A) at position 16256) downstream primer

<400> 7

tcctagtggg tgaggggtga 20

<210> 8

<211> 20

<212> DNA

<213> Artificial

<220>

<223> Human mtDNA (244 bp fragment of region I, 16,031-16,275, carrying guanosine nucleotide (G) at position 16256) downstream primer

<400> 8

tcctagtggg tgaggggtgg 20

<210> 9

<211> 20

<212> DNA

<213> Artificial

<220>

<223> Human mtDNA (244 bp fragment of region I, 16,031-16,275, carrying thyrosine nucleotide (T) at position 16256) downstream primer

<400> 9

tcctagtggg tgaggggtgt 20

<210> 10

<211> 20

<212> DNA

<213>  Artificial

<220>
<223>  Human mtDNA (244 bp fragment of region I, 16,031-16,275, carrying cytosine nucleotide (C) at position 16256) downstream primer

<400>  10
tcctagtggg tgaggggtgc                                                    20

<210>  11
<211>  20
<212>  DNA
<213>  Artificial

<220>
<223>  Human mtDNA downstream primer

<400>  11
gataaataat aggatgagga                                                    20

<210>  12
<211>  20
<212>  DNA
<213>  Artificial

<220>

<223> Human mtDNA downstream primer

<400> 12

gataaataat aggatgaggg 20

<210> 13

<211> 20

<212> DNA

<213> Artificial

<220>

<223> Human mtDNA downstream primer

<400> 13

gataaataat aggatgaggt 20

<210> 14

<211> 20

<212> DNA

<213> Artificial

<220>

<223> Human mtDNA downstream primer

<400> 14

gataaataat aggatgaggc 20

<210> 15
<211> 23
<212> DNA
<213> Artificial

<220>
<223> Homo sapiens Cytochrome b-245 beta upstream primer

<400> 15
ctccctctga atattttgtt atc 23

<210> 16
<211> 21
<212> DNA
<213> Artificial

<220>
<223> Homo sapiens Cytochrome b-245 beta downstream primer

<400> 16
gaccaccttc tgttgagatc a 21

<210> 17
<211> 21

<212> DNA

<213> Artificial

<220>

<223> Homo sapiens Cytochrome b-245 beta downstream primer

<400> 17

gaccaccttc tgttgagatc g 21

<210> 18

<211> 21

<212> DNA

<213> Artificial

<220>

<223> Homo sapiens Cytochrome b-245 beta downstream primer

<400> 18

gaccaccttc tgttgagatc t 21

<210> 19

<211> 21

<212> DNA

<213> Artificial

<220>

<223> Homo sapiens Cytochrome b-245 beta downstream primer

<400> 19
gaccaccttc tgttgagatc c 21

<210> 20
<211> 1390
<212> DNA
<213> Escherichia coli

<400> 20
ctggcgaaag ggggatgtgc tgcaaggcga ttaagttggg taacgccagg
gttttcccag 60

tcacgacgtt gtaaaacgac ggccagtgaa tccgtaatca tggtcatagc
tgtttcctgt 120

gtgaaattgt tatccgctca caattccaca caacatacga gccggaagca
taaagtgtaa 180

agcctggggt gcctaatgag tgagctaact cacattaatt gcgttgcgct
cactgcccgc 240

tttccagtcg ggaaacctgt cgtgccagct gcattaatga atcggccaac
gcgcggggag 300

aggcggtttg cgtattgggc gccagggtgg tttttctttt caccagtgag
acgggcaaca    360

gctgattgcc cttcaccgcc tggccctgag agagttgcag caagcggtcc
acgctggttt    420

gccccagcag gcgaaaatcc tgtttgatgg tggttaacgg cgggatataa
catgagctgt    480

cttcggtatc gtcgtatccc actaccgaga tatccgcacc aacgcgcagc
ccggactcgg    540

taatggcgcg cattgcgccc agcgccatct gatcgttggc aaccagcatc
gcagtgggaa    600

cgatgccctc attcagcatt tgcatggttt gttgaaaacc ggacatggca
ctccagtcgc    660

cttcccgttc cgctatcggc tgaatttgat tgcgagtgag atatttatgc
cagccagcca    720

gacgcagacg cgccgagaca gaacttaatg ggcccgctaa cagcgcgatt
tgctggtgac    780

ccaatgcgac cagatgctcc acgcccagtc gcgtaccgtc ttcatgggag
aaaataatac    840

tgttgatggg tgtctggtca gagacatcaa gaaataacgc cggaacatta
gtgcaggcag 900

cttccacagc aatggcatcc tggtcatcca gcggatagtt aatgatcagc
ccactgacgc 960

gttgcgcgag aagattgtgc accgccgctt tacaggcttc gacgccgctt
cgttctacca 1020

tcgacaccac cacgctggca cccagttgat cggcgcgaga tttaatcgcc
gcgacaattt 1080

gcgacggcgc gtgcagggcc agactggagg tggcaacgcc aatcagcaac
gactgtttgc 1140

ccgccagttg ttgtgccacg cggttgggaa tgtaattcag ctccgccatc
gccgcttcca 1200

ctttttcccg cgttttcgca gaaacgtggc tggcctggtt caccacgcgg
gaaacggtct 1260

gataagagac accggcatac tctgcgacat cgtataacgt tactggtttc
acattcacca 1320

ccctgaattg actctcttcc gggcgctatc atgccatacc gcgaaaggtt
ttgcgccatt   1380
cgatggtgtc   1390

<210>  21
<211>  2743
<212>  DNA
<213>  Artificial

<220>
<223>  pGEM*-3Zvector secuence  (Promega Corp.)

<400>  21
gggcgaattc gagctcggta cccggggatc ctctagagtc gacctgcagg
catgcaagct     60

tgagtattct atagtgtcac ctaaatagct tggcgtaatc atggtcatag
ctgtttcctg    120

tgtgaaattg ttatccgctc acaattccac acaacatacg agccggaagc
ataaagtgta    180

aagcctgggg tgcctaatga gtgagctaac tcacattaat tgcgttgcgc
tcactgcccg    240

ctttccagtc gggaaacctg tcgtgccagc tgcattaatg aatcggccaa
cgcgcgggga    300

gaggcggttt gcgtattggg cgctcttccg cttcctcgct cactgactcg
ctgcgctcgg    360

tcgttcggct gcggcgagcg gtatcagctc actcaaaggc ggtaatacgg
ttatccacag    420

aatcagggga taacgcagga aagaacatgt gagcaaaagg ccagcaaaag
gccaggaacc    480

gtaaaaaggc cgcgttgctg gcgtttttcc ataggctccg cccccctgac
gagcatcaca    540

aaaatcgacg ctcaagtcag aggtggcgaa acccgacagg actataaaga
taccaggcgt    600

ttccccctgg aagctccctc gtgcgctctc ctgttccgac cctgccgctt
accggatacc    660

tgtccgcctt tctcccttcg ggaagcgtgg cgctttctca tagctcacgc
tgtaggtatc    720

tcagttcggt gtaggtcgtt cgctccaagc tgggctgtgt gcacgaaccc
cccgttcagc    780

ccgaccgctg cgccttatcc ggtaactatc gtcttgagtc caacccggta agacacgact 840

tatcgccact ggcagcagcc actggtaaca ggattagcag agcgaggtat gtaggcggtg 900

ctacagagtt cttgaagtgg tggcctaact acggctacac tagaagaaca gtatttggta 960

tctgcgctct gctgaagcca gttaccttcg gaaaaagagt tggtagctct tgatccggca 1020

aacaaaccac cgctggtagc ggtggttttt ttgtttgcaa gcagcagatt acgcgcagaa 1080

aaaaaggatc tcaagaagat cctttgatct tttctacggg gtctgacgct cagtggaacg 1140

aaaactcacg ttaagggatt ttggtcatga gattatcaaa aaggatcttc acctagatcc 1200

ttttaaatta aaaatgaagt tttaaatcaa tctaaagtat atatgagtaa acttggtctg 1260

acagttacca atgcttaatc agtgaggcac ctatctcagc gatctgtcta tttcgttcat 1320

ccatagttgc ctgactcccc gtcgtgtaga taactacgat acgggagggc ttaccatctg 1380

gccccagtgc tgcaatgata ccgcgagacc cacgctcacc ggctccagat ttatcagcaa 1440

taaaccagcc agccggaagg gccgagcgca gaagtggtcc tgcaacttta tccgcctcca 1500

tccagtctat taattgttgc cgggaagcta gagtaagtag ttcgccagtt aatagtttgc 1560

gcaacgttgt tgccattgct acaggcatcg tggtgtcacg ctcgtcgttt ggtatggctt 1620

cattcagctc cggttcccaa cgatcaaggc gagttacatg atcccccatg ttgtgcaaaa 1680

aagcggttag ctccttcggt cctccgatcg ttgtcagaag taagttggcc gcagtgttat 1740

cactcatggt tatggcagca ctgcataatt ctcttactgt catgccatcc gtaagatgct 1800

tttctgtgac tggtgagtac tcaaccaagt cattctgaga atagtgtatg cggcgaccga 1860

gttgctcttg cccggcgtca atacgggata ataccgcgcc acatagcaga actttaaaag 1920

tgctcatcat tggaaaacgt tcttcggggc gaaaactctc aaggatctta ccgctgttga 1980

gatccagttc gatgtaaccc actcgtgcac ccaactgatc ttcagcatct tttactttca 2040

ccagcgtttc tgggtgagca aaaacaggaa ggcaaaatgc cgcaaaaaag ggaataaggg 2100

cgacacggaa atgttgaata ctcatactct tcctttttca atattattga agcatttatc 2160

agggttattg tctcatgagc ggatacatat ttgaatgtat ttagaaaaat aaacaaatag 2220

gggttccgcg cacatttccc cgaaaagtgc cacctgacgt ctaagaaacc attattatca 2280

tgacattaac ctataaaaat aggcgtatca cgaggccctt tcgtctcgcg cgtttcggtg 2340

atgacggtga aaacctctga cacatgcagc tcccggagac ggtcacagct tgtctgtaag 2400

cggatgccgg gagcagacaa gcccgtcagg gcgcgtcagc gggtgttggc gggtgtcggg 2460

gctggcttaa ctatgcggca tcagagcaga ttgtactgag agtgcaccat atgcggtgtg 2520

aaataccgca cagatgcgta aggagaaaat accgcatcag gcgccattcg ccattcaggc 2580

tgcgcaactg ttgggaaggg cgatcggtgc gggcctcttc gctattacgc cagctggcga 2640

aagggggatg tgctgcaagg cgattaagtt gggtaacgcc agggttttcc cagtcacgac 2700

gttgtaaaac gacggccagt gaattgtaat acgactcact ata 2743

<210> 22

<211> 20

<212> DNA

<213> Artificial

<220>

<223> Reverse primer used in LacI based Assay

<400> 22

gacaccatcg aatggtgcaa 20

<210> 23

<211> 21

<212> DNA

<213> Artificial

<220>

<223> Forward primer used in LacI based Assay

<400> 23

ctggcgaaag ggggatgtgc t 21

<210> 24

<211> 20

<212> DNA

<213> Artificial

<220>

<223> Beta - lactamase region reverse primer

<400> 24
tttcacaccg catatggtgc 20

<210> 25
<211> 26
<212> DNA
<213> Artificial

<220>
<223> Beta - lactamase forward primer

<400> 25
aagtatgcat gagtaaactt ggtctg 26

<210> 26
<211> 25
<212> DNA
<213> Artificial

<220>
<223> p3ZIq24 origin region reverse primer

<400> 26
actcatgcat actttagatt gattt 25

<210> 27

<211> 18

<212> DNA

<213> Artificial

<220>

<223> p3ZIq24 origin region forward primer

<400> 27

agagtgcacc atatgcgg 18

## Description

### Field of the Invention.

The present invention relates to the field of molecular biology. The method of the invention is useful to increase both the specificity and the fidelity of PCR or other assays that employ polymerases.

### Background of the Invention.

Detecting and identifying variations in DNA sequences among individuals and species has provided insights into evolutionary relationships, inherited disorders, acquired disorders and others aspects of molecular genetic and medicine.

These variations may involve different lengths of DNA, from several nucleotides down to just a single one. The detection of single nucleotide polymorphisms (SNPs) is a challenging task aimed to provide new developments in the field of Molecular Biology.

The analysis of sequence variation has traditionally been performed by restriction fragment length polymorphism (RFLP) in a Southern blot format, or more recently, by digesting PCR products. The RFLP analyses are based on a change in the restriction fragment length as a result of a change in the sequence. Nowadays most techniques rely on the differential annealing of allele-specific oligonucleotides to a template. Some of these techniques are allele-specific oligonucleotide hybridization (ASO), reverse dot blot, competitive oligonucleotide priming (COP), primer extension sequence test (PEST), nucleic acid depolymerization (READIT®), and amplification refractory mutation system (ARMS), also known as allele-specific PCR (ASP), PCR amplification of specific alleles (PASA) and allele-specific amplification (ASA).

A key aspect of the methods that are based upon oligonucleotide base-pairing is that the allele-specific oligonucleotide must anneal only to the homologous sequence to prevent misleading results. However, this is not always the case with the methods where 3' mismatches are used to identify the different alleles. Newton et al.(Nucleic Acids Res. 17: 2503, 1898) and Kwok, et al. (Nucleic Acids Res. 18: 999, 1990) report that a 3' terminal mismatch on the PCR primer produced variable results, making it necessary to add a 3' terminal mismatch accompanied by a second mismatch within the last four nucleotides of the primer. The arbitrariness in the addition of extra mismatches near the 3' end on individual primers in every particular instance limits the general application of the technique in a simple and universal fashion. Also, because of the lower selectivity due to the formation of false DNA synthesis products when using single 3' mismatch primers, the informative power of the gene variation analyses and gene mutation analyses is limited. The formation of false DNA synthesis products can lead to false findings, as a result of which concerning risks arise for the patient and for biomedical research in general.

The patent US 6.403.313, teaches methods to detect specific hybridization between single-stranded probes and non-denatured double-stranded targets to form triplex by an intercalating agent, thus obviating the need to denature the target. This method can be used to determine the number of mismatched pairs in a hybridization complex, and to map genomes.

Bodmer et.al. (WO 01/75155), teach methods that can distinguish between specific and non-specific amplification products, for example adding to the post-amplification products an amount of small molecules sufficient to increase the pH of the sample products, wherein the pH is 11-14 and then assaying the post-amplification sample product in order to detect and/or quantify any double-stranded nucleic acid present. The method is useful for detecting and/or quantifying a specific double-stranded nucleic acid amplification product in a nucleic amplification reaction post-amplification sample, as in ARMS-PCR methods for SNP typing.

U. S. pat N° 5,639,611 discloses an allele specific PCR reaction with two primers (mutant and normal alleles), which one of the primers is complementary to the first allele, but which primer forms a mismatch with the second allele at the 3' end of the primer, employing a DNA polymerase wherein the first allele is specifically amplified but little or no amplification the second allele occurs.

U. S. Patent application 0010009761 disclose a method for detecting a single nucleotide polymorphism in a target by isothermal nucleic acid amplification, hybridizing a detector primer to the target wherein the detector primer comprises a diagnostic nucleotide for the single nucleotide polymorphism about one to four nucleotides from 3' terminal nucleotide of the detector primer, which is complementary to the target sequence, amplifying the target, determining an efficiency of detector primer extension and detecting de presence or absence of the single nucleotide polymorphism based on the efficiency of detector primer extension. This application disclosed the ARMS method.

U. S. Patent N° 6.312.894 disclosed an hybridization and mismatch discrimination using oligonucleotides conjugated to minor grooves binders. The minor grooves binders is a molecule having a molecular weight of approximately 150 to 2,000 Daltons as 1,2-dihydro-(3H)-pyrrolo[3,2-e]indole-7-carboxylate that binds in a non-intercalating manner into de minor groove of a double-stranded nucleic acid.

On the other hand, the use of high fidelity DNA polymerases in the polymerase chain reaction (PCR) is essential for reducing errors in the amplification of products that are intended for cloning, sequencing and expression. Several DNA Polymerases with 3'-5' exonuclease-proofreading activity *(Pfu, Vent, Deep Vent)* are currently used for such high fidelity applications. However, *Tag* DNA polymerase, which is the most used enzyme in routine PCR, does not have a proofreading activity and shows a fairly low fidelity (Tindall, K.R. and Kunkel, T.A. (1988) *Biochemistry* 28: 6008). Some improvements on the fidelity of *Taq* DNA polymerase have been reported using pH 5-6 or equimolar concentrations of $MgCl_2$ and dNTPs (Eckert, K.A. and Kunkel, T.A. (1990) *Nucl.Acids.Res.* 18:3739).

It would be therefore convenient to have a method to increase the selectivity of gene variation analyses and, by suppressing the formation of false positives, to prevent wrong diagnoses and erroneous findings. The goal of the present invention is to increase the selectivity in specific nucleic acids sequence analyses adding an intercalating agent to the conventional reactions medium, reducing the efficiency of primer extension by polymerase when the 3' end of a primer does not hybridize perfectly with the target sequence. The addition of the intercalating agent avoids the need to place a second mismatch in the sequence of the detector primer which is not directed to detection or identification of the allele of interest. Since the priming selectivity repeats in every following nucleotide addition to the growing chain, it is also another embodiment of the present invention to increase the fidelity of the amplification of nucleic acid fragments, in particular, those which will be used in cloning, expression and all other applications where overall low error rates in nucleic acid chains may be desired.

**Summary of the Invention.**

It is therefore an object of the present invention to provide a method for increasing the selectivity of primer extension by polymerase enzymes when the 3' end of the initial primer or the growing, priming strand does not hybridize perfectly with the target, increasing the selectivity of single nucleotide mutation or gene analyses by suppressing false positive results and enhancing the fidelity of the polymerase reaction by avoiding the incorporation of mispairs, comprising the steps of:

(a) obtaining a nucleic acid sample;
(b) hybridizing said nucleic acid sample to a primer;
(c) subjecting said nucleic acid sample hybridized to a extension reaction by extending the primer with a polymerizing enzyme, wherein the reaction extension mixture medium contains an intercalating agent; and
(d) detecting the presence of extension products.

In a preferred embodiment the methods comprises: (a) obtaining a nucleic acid sample; (b) hybridizing said nucleic acid sample to primer pair by subjecting said nucleic acid sample hybridized to a PCR, wherein the PCR reaction mixture contains an intercalating agent; and (c) detecting the presence of amplification products

The intercalating agent may be any intercalating agent as ethidium bromide, dihydroethidium, ethidium homodimer-1, ethidium homodimer-2, acridine, propidium iodide, YOYO®-1, TOTO®-1, or any other flat organic molecule capable of stacking between the nucleic acid bases. In a preferred embodiment the intercalating agent is ethidium bromide at a concentration about 1 to 7 µg/ml.

The methods of the present invention decrease the amount of extension products when the 3' end of a primer or growing nucleic acid chain does not hybridize perfectly with the target sequence, increase the selectivity detection of single nucleotide mutation and/or suppress false positive extension products in gene analyses and/or decrease the error rate in PCR amplifications.

It is still another object of the present invention to provide a reaction extension mixture reagent, wherein the reagent comprises: a polymerizing enzyme, dNTPs, a buffer, an intercalating agent as for example ethidium bromide, dihydroethidium, ethidium homodimer-1, ethidium homodimer-2, acridine, propidium iodide, YOYO®-1, TOTO®-1, or any other flat organic molecule capable of stacking between the nucleic acid bases.

The reaction extension mixture reagent may be a PCR reaction mixture or any medium which an extension reaction of nucleic acid was made.

The above and other objects, features and advantages of this invention will be better understood when taken in connection with the accompanying drawings and description.

**Brief description of the drawings.**

The present invention is illustrated by way of example in the following drawings wherein:

FIG. 1 shows the amplification results on mtDNA in 2% agarose gel stained with ethidium bromide carrying a T to C transition at position 16311 using primers FW I (SEQ ID N° 1) and either 16311A (SEQ ID N° 3), 16311G (SEQ ID N° 4), 16311T (SEQ ID N° 5) or 16311C (SEQ ID N° 6) at different ethidium bromide concentrations.

Lane 1 through 4: 16311A (SEQ ID N° 3), 16311G (SEQ ID N° 4), 16311T (SEQ ID N° 5) and 16311C (SEQ ID N° 6) without ethidium bromide. Lane 5 through 8: 16311A (SEQ ID N° 3), 16311G (SEQ ID N° 4), 16311T (SEQ ID N° 5) and 16311C (SEQ ID N° 6) with 4.5 µg/ml ethidium bromide. Lane 9 through 12: 16311A (SEQ ID N° 3), 16311G (SEQ ID N° 4), 16311T (SEQ ID N° 5) and 16311C (SEQ ID N° 6) with 5.0 µg/ml ethidium bromide. Lane 13 through 16: 16311A (SEQ ID N° 3), 16311G (SEQ ID N° 4), 16311T (SEQ ID N° 5) and 16311C (SEQ ID N° 6) C with 5.5 µg/ml ethidium bromide. Lane 16 through 20: 16311A (SEQ ID N° 3), 16311G (SEQ ID N° 4), 16311T (SEQ ID N° 5) and 16311C (SEQ ID N° 6) with 6.0 µg/ml ethidium bromide. Lane 21: 100 bp ladder (Promega Corp.);

FIG. 2 shows the amplification results on mtDNA in 2% agarose gel stained with ethidium bromide showing the amplification results on mtDNA carrying an Andersons' T nucleotide at position 16311 using primers FW I and either 16311A (SEQ ID N° 3), 16311G (SEQ ID N° 4), 16311T (SEQ ID N° 5) or 16311C (SEQ ID N° 6) at different ethidium bromide concentrations.

Lane 1 through 4: 16311A (SEQ ID N° 3), 16311G (SEQ ID N° 4), 16311T (SEQ ID N° 5) and 16311C (SEQ ID N° 6) without ethidium bromide. Lane 5 through 8: 16311A (SEQ ID N° 3), 16311G (SEQ ID N° 4), 16311T (SEQ ID N° 5) and 16311C (SEQ ID N° 6) with 4.5 µg/ml ethidium bromide. Lane 9 through 12: 16311A (SEQ ID N° 3), 16311G (SEQ ID N° 4), 16311T (SEQ ID N° 5) and 16311C (SEQ ID N° 6) with 5.0 µg/ml ethidium bromide. Lane 13 through 16: 16311A (SEQ ID N° 3), 16311G (SEQ ID N° 4), 16311T (SEQ ID N° 5) and 16311C (SEQ ID N° 6) with 5.5 µg/ml ethidium bromide. Lane 16 through 20: 16311A (SEQ ID N° 3), 16311G (SEQ ID N° 4), 16311T (SEQ ID N° 5) and 16311C (SEQ ID N° 6) with 6.0 µg/ml ethidium bromide. Lane 21: 100 bp ladder (Promega Corp);

FIG. 3 shows the amplification results on mtDNA in a 2% agarose gel stained with ethidium bromide showing the amplification results on mtDNA carrying a C nucleotide at position 16256 using primers FW I and either 16256A (SEQ ID N° 7), 16256G (SEQ ID N° 8), 16256T (SEQ ID N° 9) or 16256C (SEQ ID N° 10) at different ethidium bromide concentrations.

Lane 1 through 4: 16256A (SEQ ID N° 7), 16256G (SEQ ID N° 8), 16256T (SEQ ID N° 9) and 16256C (SEQ ID N° 10) without ethidium bromide. Lane 5 through 8: 16256A (SEQ ID N° 7), 16256G (SEQ ID N° 8), 16256T (SEQ ID N° 9) and 16256C (SEQ ID N° 10) with 4.5 µg/ml ethidium bromide. Lane 9 through 12: 16256A (SEQ ID N° 7), 16256G (SEQ ID N° 8), 16256T (SEQ ID N° 9) and 16256C (SEQ ID N° 10) with 5.0 µg/ml ethidium bromide. Lane 13 through 16: 16256A (SEQ ID N° 7), 16256G (SEQ ID N° 8), 16256T (SEQ ID N° 9) and 16256C (SEQ ID N° 10) with 5.5 µg/ml ethidium bromide. Lane 16 through 20: 16256A (SEQ ID N° 7), 16256G (SEQ ID N° 8), 16256T (SEQ ID N° 9) and 16256C (SEQ ID N° 10) with 6.0 µg/ml ethidium bromide. Lane 21: 100 bp ladder (Promega Corp.);

FIG. 4 shows the amplification results on mtDNA in 2% agarose gel stained with ethidium bromide showing the amplification results on mtDNA carrying a G nucleotide at position 143 using primers FW II (SEQ ID N° 2) and either 143A (SEQ ID N° 11), 143G (SEQ ID N° 12), 143T (SEQ ID N° 13) or 143C (SEQ ID N° 14) at different ethidium bromide concentrations.

Lane 1 through 4: 143A (SEQ ID N° 11), 143G (SEQ ID N° 12), 143T (SEQ ID N° 13) and 143C (SEQ ID N° 14) without ethidium bromide. Lane 5 through 8: 143A (SEQ ID N° 11), 143G (SEQ ID N° 12), 143T (SEQ ID N° 13) and 143C (SEQ ID N° 14) with 4.5 µg/ml ethidium bromide. Lane 9 through 12: 143A (SEQ ID N° 11), 143G (SEQ ID N° 12), 143T (SEQ ID N° 13) and 143C (SEQ ID N° 14) with 5.0 µg/ml ethidium bromide. Lane 13 through 16: 143A (SEQ ID N° 11), 143G (SEQ ID N° 12), 143T (SEQ ID N° 13) and 143C (SEQ ID N° 14) with 5.5 µg/ml ethidium bromide. Lane 16 through 20: 143A (SEQ ID N° 11), 143G (SEQ ID N° 12), 143T (SEQ ID N° 13) and 143C (SEQ ID N° 14) with 6.0 µg/ml ethidium bromide. Lane 21: 100 bp ladder (Promega Corp.)

FIG. 5 shows the amplification results on mtDNA in 2% agarose gel stained with ethidium bromide showing the amplification results on mtDNA carrying a T to C transition at position 16311 with 5.0 µg/ml ethidium bromide using primers FW I and either 16311A (SEQ ID N° 3) or 16311G (SEQ ID N° 4) at different concentrations of downstream primers.

Lane 1 through 2: 100 pmole of 16311A (SEQ ID N° 3) and 16311G (SEQ ID N° 4) and 5.0 µg/ml ethidium bromide. Lane 3 through 4: 50 pmole 16311A (SEQ ID N° 3) and 16311G (SEQ ID N° 4), 5.0 µg/ml ethidium bromide. Lane 5 through 6: 5 pmole 16311A (SEQ ID N° 3) and 16311G (SEQ ID N° 4), 5.0 µg/ml of ethidium bromide. Lane 7 through 8: 2.5 pmole 16311A (SEQ ID N° 3) (SEQ ID N° 3) and 16311G (SEQ ID N° 4), 5.0 µg/ml ethidium bromide. Lane 9 through 10: 0.5 pmole 16311A (SEQ ID N° 3), (SEQ ID N° 3) and 16311G (SEQ ID N° 4), 5.0 µg/ml ethidium bromide. Lane 11: 100 bp ladder (Promega Corp.). Lane 13 through 14: 100 pmole 16311A (SEQ ID N° 3) and 16311G (SEQ ID N° 4), without ethidium bromide. Lane 15 through 16: 50 pmole 16311A (SEQ ID N° 3) and 16311G (SEQ ID N° 4), without ethidium bromide. Lane 17 through 18: 5 pmole 16311A (SEQ ID N° 3) and 16311G (SEQ ID N° 4), without ethidium bromide. Lane 19 through 20: 2.5 pmole 16311A (SEQ ID N° 3), and 16311G (SEQ ID N° 4), without ethidium bromide. Lane 21 through 22: 0.5 pmole 16311A (SEQ ID N° 3) and 16311G (SEQ ID N° 4), without ethidium bromide. Lane 23: 100 bp ladder (Promega Corp.).

FIG. 6 shows the amplification results on mtDNA in 2% agarose gel stained with ethidium bromide showing the amplification results on mtDNA carrying a T to C transition at position 16311 with 5.0 µg/ml ethidium bromide using primers FW I and either 16311A (SEQ ID N° 3) or 16311G (SEQ ID N° 4) at different concentrations of template.

Lane 1 and 2: 16311A (SEQ ID N° 3) and 16311G (SEQ ID N° 4) at 500 ng total DNA, 5.0 µg/ml ethidium bromide. Lane 3 and 4: 16311A (SEQ ID N° 3) and 16311G (SEQ ID N° 4) at 50 ng total DNA, 5.0 µg/ml ethidium bromide. Lane 5 and 6: 16311A (SEQ ID N° 3) and 16311G (SEQ ID N° 4) at 10 ng total DNA, 5.0 µg/ml ethidium bromide. Lane 7 and 8: 16311A (SEQ ID N° 3) and 16311G (SEQ ID N° 4) at 2 ng total DNA, 5.0 µg/ml ethidium bromide. Lane 9 and 10: 16311A (SEQ ID N° 3) and 16311G (SEQ ID N° 4) at 0.5 ng total DNA, 5.0 µg/ml ethidium bromide. Lane 11: 100 bp ladder (Promega Corp.). Lane 13 and 14: 16311A (SEQ ID N° 3) and 16311G (SEQ ID N° 4) at 500 ng total DNA, without ethidium bromide. Lane 15 and 16: 16311A (SEQ ID N° 3) and 16311G (SEQ ID N° 4) at 50 ng total DNA, without ethidium bromide. Lane 17 and 18: 16311A (SEQ ID N° 3) and 16311G (SEQ ID N° 4) at 10 ng total DNA, without ethidium bromide. Lane 19 and 20: 16311A (SEQ ID N° 3) and 16311G (SEQ ID N° 4) at 2 ng total DNA, without ethidium bromide. Lane 21 and 22: 16311A (SEQ ID N° 3) and 16311G (SEQ ID N° 4) at 0.5 ng total DNA, without ethidium bromide. Lane 23: 100 bp ladder (Promega Corp.).

FIG. 7 shows the amplification results on mtDNA in 2% agarose gel stained with ethidium bromide showing the amplification results on mtDNA carrying a T to C transition at position 16311 with primers FW I and either 16311A (SEQ ID N° 3) or 16311G (SEQ ID N° 4) adding 5.0 µg/ml ethidium bromide before, in between and after the primers and the template.

Lane 1 and 2: 16311A (SEQ ID N° 3) and 16311G (SEQ ID N° 4) with 5.0 µg/ml ethidium bromide added after the primers and the template. Lane 3 and 4: 16311A (SEQ ID N° 3) and 16311G (SEQ ID N° 4) with 5.0 µg/ml ethidium bromide added after the template and before the primers. Lane 5 and 6: 16311A (SEQ ID N° 3) and 16311G (SEQ ID N° 4) with 5.0 µg/ml ethidium bromide added before the primers and the primers template. Lane 7 and 8: 16311A (SEQ ID N° 3) and 16311G (SEQ ID N° 4) with 5.0 µg/ml ethidium bromide added after the primers and before the template. Lane 9: 100 bp ladder (Promega Corp.). Lane 10 and 11: 16311A (SEQ ID N° 3) and 16311G (SEQ ID N° 4) with water added after the primers and the template. Lane 12 and 13: 16311A (SEQ ID N° 3) and 16311G (SEQ ID N° 4) with water added after the template and before the primers. Lane 14 and 15: 16311A (SEQ ID N° 3) and 16311G (SEQ ID N° 4) with water added before the primers and the primers template. Lane 16 and 17: 16311A (SEQ ID N° 3) and 16311G (SEQ ID N° 4) with water added after the primers and before the template. Lane 18: 100 bp ladder (Promega Corp.).

FIG. 8 shows the amplification results on mtDNA in 2% agarose gel stained with ethidium bromide showing the Pfu DNA Polymerase amplification results on mtDNA carrying a T to C transition at position 16311 using primers FW I (SEQ ID N° 1) and either 16311A (SEQ ID N° 3), 16311G (SEQ ID N° 4), 16311T (SEQ ID N° 5) or 16311C (SEQ ID N° 6) at different ethidium bromide concentrations.

Lane 1 through 4: 16311A (SEQ ID N° 3), 16311G (SEQ ID N° 4), 16311T (SEQ ID N° 5) and 16311C (SEQ ID N° 6) without ethidium bromide. Lane 5 through 8: 16311A (SEQ ID N° 3), 16311G (SEQ ID N° 4), 16311T (SEQ ID N° 5) and 16311C (SEQ ID N° 6) with 4.5 µg/ml ethidium bromide. Lane 9: 100 bp ladder (Promega Corp.). Lane 10 through 13: 16311A (SEQ ID N° 3), 16311G (SEQ ID N° 4), 16311T (SEQ ID N° 5) and 16311C (SEQ ID N° 6) with 5.0 µg/ml ethidium bromide. Lane 14 through 17: 16311A (SEQ ID N° 3), 16311G (SEQ ID N° 4), 16311T (SEQ ID N° 5) and 16311C (SEQ ID N° 6) with 5.5 µg/ml ethidium bromide. Lane 18: 100 bp ladder (Promega Corp.).

FIG. 9 shows the amplification results on genomic DNA from human patient carrying a C to T transition at position 1785 of the exon 8 of Homo sapiens cytochrome b-245 beta polypeptide (CYBB) gene and from an individual control in 2% agarose gel stained with ethidium bromide. DNA samples were amplified with or without ethidium bromide in four separate reactions sharing the upstream primer CYBB8FW (SEQ ID N° 15) and having either of the four alternative downstream primers (CYBB8A (SEQ ID N°: 16), CYBB8G (SEQ ID N°: 17), CYBB8T (SEQ ID N°: 18) and CYBB8C (SEQ ID N°: 19)).

Lane 1 through 4: CYBB8A (SEQ ID N°: 16), CYBB8G (SEQ ID N°: 17), CYBB8T (SEQ ID N°: 18) and CYBB8C (SEQ ID N°: 19) without ethidium bromide, patient DNA. Lane 5 through 8: CYBB8A (SEQ ID N°: 16), CYBB8G (SEQ ID N°: 17), CYBB8T (SEQ ID N°: 18) and CYBB8C (SEQ ID N°: 19) without ethidium bromide, control DNA. Lane 9: 100 bp ladder (Promega Corp.). Lane 10 through 13: CYBB8A (SEQ ID N°: 16), CYBB8G (SEQ ID N°: 17), CYBB8T (SEQ ID N°: 18) and CYBB8C (SEQ ID N°: 19) with 5.0 µg/ml ethidium bromide, patient DNA. Lane 14 through 17: CYBB8A (SEQ ID N°: 16), CYBB8G (SEQ ID N°: 17), CYBB8T (SEQ ID N°: 18) and CYBB8C (SEQ ID N°: 19) with 5.0 µg/ml ethidium bromide, control DNA. Lane 18: 100 bp ladder (Promega Corp.).

FIG. 10-a: Shows a scheme of the p3ZIQ24 construct

FIG. 10-b: Shows the 2,482 bp amplification product of the lacI gene amplification in 0.7% agarose gel stained with ethidium bromide. DNA samples were amplified with or without ethidium bromide in seven separate reactions using the forward primer Orifw (SEQ ID N° 27) and the reverse primer OriRv (SEQ ID N°: 26).

Lanes 1 through 3: without ethidium bromide.

Lanes 4 through 6: with 2.0 µg/ml ethidium bromide. Lane 7: with 3.0 µg/ml ethidium bromide.

Lane 8: 1 Kb ladder (Promega Corp.).

**Description of the preferred embodiments.**

Definitions:

As use herein, the term "intercalating agent" refers a moiety that is able to intercalate between the bases of a nucleic acid molecule.

As use herein, the term "transition" is the substitution in DNA or RNA of one purine by another purine, or of one pyrimidine by another pyrimidine.

As use herein, the term "primer" or "priming strand" refers either to an oligonucleotide sequence that is added to the reaction extension mixture wherein such oligonucleotide sequence hybridizes with a target sequence on the nucleic acid template, necessary for the beginning of the nucleic acid chain extension reaction or to the growing nucleic acid chain so that whenever a nucleotide is added to the growing chain, this growing chain with the new nucleotide added acts as a primer for the addition of the next nucleotide, and so on.

The present invention provides methods for increasing the fidelity and selectivity of primer or chain extension for polymerases in the presence of an intercalating agent as ethidium bromide when the 3' end of a primer (added primer or growing chain acting as a primer as described above) does not hybridized perfectly with the target sequence. It provides a method for detecting and identifying sequence variation in a nucleic acid by primer extension of polymerases and can be adapted for use as a means for distinguishing or identifying the nucleotide in the target sequence, which is at the site where the mismatch between the primer and the target occurs.

Moreover the present invention also provides a method to increase the fidelity of *Tag* DNA polymerase by adding ethidium bromide and therefore can be adapted for its use in cloning, expression and all other applications where low error rate may be desired. The fidelity and efficiency improvements may also be well suited for other DNA or RNA polymerase enzymes and for different nucleic acid templates.

The efficiency of primer extension is detected as an indication of the presence and/or identity of the sequence variation in the target. The methods are particularly well suited for detecting and identifying single nucleotide differences between a target sequence of interest, for example a mutant allele of the gene, and a second nucleic acid sequence, for example a wild type allele for the same gene.

Addition of the intercalating agent for example ethidium bromide to an extension reaction mixture or hybridization medium, preferably a PCR reaction mixture improves the selectivity of sequence-specific analyses by decreasing 3'-mismatch in priming and extension of the growing chain. The methods of the invention increase the selectivity of gene variation analyses and suppress the formation of false positive to prevent wrong diagnoses and findings. The method of the invention may be used in any assay that employs an extension step of a target nucleic acid sequence, wherein the 3' end of the added primer does not hybridized perfectly with that target nucleic acid sequence and/or when low error rate products may be desired.

The extension reaction mixture or hybridization media can be any conventional medium known to be suitable for the extension reaction, for example the liquid medium can comprise nucleotide sequence, primers, water, buffer and salts. The extension reaction can be carried out under a wide variety of conditions, having different temperature, electrostatic strength, salt concentration and composition.

An expert knows that the concentration of the intercalating agent must be adjusted according the extension conditions and the type of intercalating agent used. In a preferred embodiment, the intercalating agent is ethidium bromide, which is present in the extension reaction mixture in a concentration about 1μg/ml and 7μg/ml.

The use of the inventive reaction mixture leads to a clearly improved sensitivity and selectivity of gene polymorphism and gene mutation analyses in animal, bacterial, plants and human genome, preventing wrong diagnoses. It also improves the obtainment of low error rate products necessary for cloning or expression assays. By these means, it is possible to carry out such detections on samples, which previously could not be analyzed in this way. Moreover, the invention leads to a dramatic reduction in the costs of detections, and is rapid and sensitive.

The inventive extension reaction mixture makes possible a distinct increase in the information power of the semi-quantitative and totally quantitative determination of gene variation in tissues and organs in healthy, diseased and medicinally affected state.

In a preferred embodiment, the method of the invention is directed to detecting single nucleotides polymorphisms (SNPs) in a nucleic acid sequence of interest, for example alleles, and to identifying such SNPs or alleles. Such nucleotide sequence variants may be detected directly in a sample to be analyzed during extension and/or amplification of the target sequence.

The inventive methods are based upon the relative inefficiency of primer extension by polymerases enzymes in the presence of an intercalating agent when there are mismatches at the 3' end of a primer hybridized to an otherwise complementary sequence. The method of the invention is useful for detecting mismatches at the 3'end when purine-pyrimidine, purine-purine or pyrimidine-pyrimidine bases mismatches are present.

The difference in the efficiency of polymerase extension (in presence of ethidium bromide) when the primer is hybridized to two different alleles may be used to indicate which allele the target nucleic acid contains. When any one of multiple alleles may be present, multiple primers are employed in the analysis, each with different potential mismatch at or near 3' end. The primer which is most efficient extended provides the identity of the allele, for example the identity of the nucleotide present in the target sequence being analyzed. If the set of primers comprising A, G, C and T at the site of allele to be identified is hybridized to the target sequence and extended, the identity of the allele will be the complement of the nucleotide in the signal primer which was most efficiently extended by the polymerase. The reaction may be performed in monoplex or multiplex format, containing either one or more sets of allelic primers.

The present invention is suitable for SNP assays and for variations that involve mismatches larger than one nucleotide. It may be applied to the currently used methods that rely on primer annealing to distinguish variations in nucleic acid. It may also be useful in the design of defined or random approaches to discover new SNPs.

The application of the invention comprises, above all, a) the pharmacogenomics, especially the discovery of genomic target for drugs candidates, b) detection of nucleotide polymorphisms, especially in molecular diagnosis of disease based on gene mutation analyses and gene polymorphisms, c) molecular diagnosis, especially the screening and diagnosis of illness relevant genes.

The inventors demonstrated that the addition of EtBr improves the specificity of DNA amplification when a 299 bp region of the human mitochondrial D-loop (16,031-16,330) carrying cytosine nucleotide (C) at position 16311 was amplified in four separate PCR reactions sharing the same upstream primer (FW I (SEQ ID N° 1)) and having either of the four 3'-end alternative downstream primers (16311A (SEQ ID N° 3), 16311G (SEQ ID N° 4), 16311T (SEQ ID N° 5) and 16311C (SEQ ID N° 6)). Increasing concentrations of ethidium bromide were added to assess the effect on the amplification of the primers carrying different 3' nucleotides. In the absence of ethidium bromide there were ambiguous results. Mismatch primer 16311A (SEQ ID N° 3) yielded about the same amount of product as the fully homologous primer 16311G (SEQ ID N° 4). There was also some product with mismatch primers 16311T (SEQ ID N° 5) and 16311C (SEQ ID N° 6). The addition of ethidium bromide at concentrations ranging from 4.5 µg/ml to 6.0 µg/ml dramatically diminished the non-specific amplification of all 3' end mismatch primers (16311A (SEQ ID N° 3), 16311C (SEQ ID N° 6) and 16311T (SEQ ID N° 5)), although had little effect on the totally complementary one (16311G (SEQ ID N° 4)) (Fig 1). Reciprocally, the same good discrimination was obtained with primer 16311A (SEQ ID N° 3) using mtDNA from a donor carrying Anderson's consensus nucleotide thymine (T) instead of cytosine (C) at position 16311 (12) (Fig 2). All mtDNA sequences were confirmed by DNA sequencing.

In order to verify if the effect of ethidium bromide was similar in other amplifications the inventors tested other regions of mtDNA. They amplified a 244 bp fragment of region I (16,031-16,275) using upstream primer FW I and downstream primers 16256A (SEQ ID N° 7), 16256G (SEQ ID N° 8), 16256T (SEQ ID N° 9) and 16256C (SEQ ID N° 10) and a 219 bp fragment of region II (16513-162) using upstream primer FW II and downstream primers 143A (SEQ ID N° 11), 143G (SEQ ID N° 12), 143T (SEQ ID N° 13) and 143C (SEQ ID N° 14). Again, the non-specific amplification of mismatch primers was abolished in all cases, regardless of the sequence being amplified (fig 3 and fig 4). As shown in fig. 4 some small variations around the optimal concentration of ethidium bromide are observed, which may be due to the different sequences of the primers.

To assess if the primer concentration can affect the selectivity brought about by the addition of ethidium bromide, the inventors tested different amounts of the otherwise hard to distinguish primers 16311A (SEQ ID N° 3) and 16311G (SEQ ID N° 4) in mtDNA carrying cytosine nucleotide (C) at position 16311. No difference in the results was found at the primer amount range tested (0.5, 2.5, 5.0, 50 and 100 pmole) (fig 5).

To verify if ethidium bromide was effective with different amounts of template, the inventors added different amounts of mtDNA carrying cytosine nucleotide (C) at position 16311 to the reaction containing either primer 16311A (SEQ ID N° 3) or 16311G (SEQ ID N° 4). The selectivity remained the same regardless of the amount of DNA template used (0.5, 2.0, 10.0, 50 and 500 ng) (fig 6) .

In order to asses if it was necessary to add the intercalating agent before the primer and the template got together in the reaction, the inventors performed PCR amplifications adding the ethidium bromide before, in between and after the addition of the primer and the template. No differences were observed in any case (fig 7).

The inventors tested the effect of ethidium bromide effect when a DNA polymerase carrying a proof-reading, 3'-5' exonuclease activity was used in the reaction instead of *Taq* DNA Polymerase. The 299 bp region of the mtDNA (16,031-16,330) carrying cytosine nucleotide (C) at position 16311 was amplified with *Pfu* DNA Polymerase (Promega) in four separate PCR reactions using primer FW I (SEQ ID N° 1) and the four alternative downstream primers (16311G (SEQ ID N° 4), 16311A (SEQ ID N° 3), 16311C (SEQ ID N° 6) and 16311T (SEQ ID N° 5)) at different ethidium bromide concentrations. Surprisingly, despite the editing activity of *Pfu* DNA Polymerase, it was obtained good specific results at a concentration range of ethidium bromide similar to the one of *Taq* (fig 8). The invention does not require an specific polymerase, any polymerase may be used to obtain the suitable product in an extension reaction.

The inventors tested the discriminatory effect of ethidium bromide on genomic DNA by amplifying a 153 bp region of the exon 8 of *Homo sapiens* cytochrome b-245 beta polypeptide (CYBB) gene (AH011465). CYBB mutations are involved in the X-linked chronic granulomatous disease (CGD) (Jirapongsananuruk, O., et. Al., *Clin. Immunol.* 104:

73, 2002, cited herein as references). DNA from a male patient having a C to T transition at nucleotide position 1785 and DNA from a male control individual were amplified with or without ethidium bromide in four separate reactions sharing the upstream primer CYBB8FW (SEQ ID N° 15) and having either of the four alternative downstream primers (CYBB8A (SEQ ID N° 16), CYBB8G (SEQ ID N° 17), CYBB8T (SEQ ID N° 18) and CYBB8C (SEQ ID N° 19)). Reactions without ethidium bromide gave a non-specific allele amplification product with the four primers in both the patient and the control. On the other hand, reactions containing ethidium bromide at 5 µg/ml yielded a distinct amplification band only with their corresponding homologous primers, CYBBA (SEQ ID N° 16) in the patient and CYBBG (SEQ ID N° 17) in the control (fig 9). This result shows that the method of the invention may be used when the template is genomic DNA.

The method of the invention can be used employing any template sequences in an extension reaction, wherein the template is genomic DNA, mitochondrial DNA, synthetic DNA or any nucleotide sequence as RNA sequences when the 3' end of the primer does not hybridized correctly with template target sequence.

In another preferred embodiment, the inventors describe an improvement in fidelity for PCR mixtures containing *Tag* DNA Polymerase in the presence of an intercalating agent, such as ethidium bromide. The fidelity was measured by using a modification of the lacI PCR mutation assay (Barnes, W.M. (1994) *Proc.Natl.Acad.Sci.* 91:2216.) described in example 7, that is based on the integrity of the repressor *lacI* (Farabaugh, P.J. (1978) *Nature* 274:765) of the Operon Lac. This modified assay, shows that an ethidium bromide concentration as low as 2 µg/ml increases the fidelity of *Tag* DNA Polymerase by almost two fold.

Throughout this application, various publications are referenced. The disclosures of all of theses publications and those references are hereby incorporated by references into this application in order to more fully describe the state of the art to which this invention pertains.

It should also be understood that the foregoing relates to preferred embodiments of the present invention and that numerous changes may be made therein without departing from the scope of the invention. The invention is further illustrated by the following examples, which are not to be construed in any way as imposing limitation upon the scope thereof. On the contrary, it is to be clearly understood that resort may be had to various other embodiments, modifications, and equivalents thereof, after reading the description herein, may suggest themselves to those skilled in the art without departing from the spirit of the present invention and/or the scope of the appended claims.

**Examples**

<u>Example 1: DNA purification and PCR amplification</u>

Total DNA was purified from fresh human blood by a salting out procedure using the Wizard® Genomic DNA Purification kit (Promega). Blood was collected in 1.5 ml microtubes containing 100 µl 0.5M EDTA as anticoagulant. Total yield of DNA was about to 10 µg for each sample.

PCR amplifications were performed in a MJ Research PTC-150 thermal cycler in a 25 µl reaction volume containing 10 mM Tris-HCl (pH 9.0), 50mM KCl, 0.1% Triton® X-100, 1.5 mM MgCl2, 200uM each deoxy-NTP, 1.5 U *Taq* DNA Polymerase (Promega Corp.), 25 pmol of upstream primer, 10 pmol of downstream primer and 10 ng of total DNA. Cycling conditions included an initial denaturalization step of 2 min. at 95°C, followed by 36 cycles of 30 sec. at 95°C, 30 sec. at 53°C and 1 min at 72°C. PCR products were electrophoresed through a 2% agarose gel and visualised with ethidium bromide.

<u>Example 2: Addition of EtBr to the PCR reaction mixture to improve the specificity of DNA amplification</u>

A 299 bp region of the human mitochondrial D-loop (16,031-16,330) carrying cytosine nucleotide (C) at position 16311 was amplified in four separate PCR reactions sharing the same upstream primer (FWD I Upstream primer 5'-ATG GGG AAG CAG ATT TGG GT-3' (SEQ ID N°1)) and having either of the four 3'-end alternative downstream primers (16311A (SEQ ID N° 3) downstream primer 5'-ACG GTA AAT GGC TTT ATG TA-3', 16311G (SEQ ID N° 4) downstream primer 5'-ACG GTA AAT GGC TTT ATG TG-3', 16311T (SEQ ID N° 5) downstream primer 5'-ACG GTA AAT GGC TTT ATG TT-3', 16311C (SEQ ID N° 6) downstream primer 5'-ACG GTA AAT GGC TTT ATG TC-3'). The PCR was carried out as in the example 1, except of increasing concentrations of ethidium bromide were added to assess the effect on the amplification of the primers carrying different 3' nucleotides. The increasing concentrations of ethidium bromide were from 4.5 to 6.0 µg/ml.

In order to verify if the effect of ethidium bromide was similar in other amplifications, we tested other regions of mtDNA. We amplified a 244 bp fragment of region I (16,031-16,275) using upstream primer FW I (showed above) and downstream primers (16256A (SEQ ID N° 7) downstream primer 5'-TCC TAG TGG GTG AGG GGT GA-3', 16256G (SEQ ID N° 8) downstream primer 5'-TCC TAG TGG GTG AGG GGT GG-3', 16256T (SEQ ID N° 9) downstream primer 5'-TCC TAG TGG GTG AGG GGT GT-3' and 16256C (SEQ ID N° 10) downstream primer 5'-TCC TAG TGG GTG AGG GGT GC-3'); and a 219 bp fragment of region II (16513-162) using upstream primer FW II (FWD II Upstream primer 5'-TCA GGG TCA TAA AGC CTA AA-3'(SEQ ID N° 2)) and downstream primers 143A (SEQ ID N° 11) downstream primer 5'-GAT AAA TAA TAG GAT GAG GA-3' , 143G (SEQ ID N° 12) downstream primer 5'-GAT AAA TAA TAG GAT GAG GG-3', 143T (SEQ ID N° 13) downstream primer 5'-GAT AAA TAA TAG GAT GAG GT-3', 143C (SEQ ID N° 14) downstream primer 5'-GAT AAA TAA TAG GAT GAG GC-3').

The PCR was carried out as in the example 1, except of increasing concentrations of ethidium bromide were added to assess the effect on the amplification of the primers carrying different 3' nucleotides in different regions of mtDNA. The increasing concentrations of ethidium bromide were from 4.5 to 6.0 μg/ml in the amplification of the 244 bp fragment and from 5.0 to 6.5 μg/ml in the amplification of the 219 bp fragment.

Example 3: The effect of primer concentration and amount of template in the methods of the invention

To assess if the primer concentration can affect the selectivity brought about by the addition of ethidium bromide, was tested different amounts of the otherwise hard to distinguish primers 16311A (SEQ ID N° 3) and 16311G (SEQ ID N° 4) (sequence showed above) in mtDNA carrying cytosine nucleotide (C) at position 16311. The PCR was carried out as in the example 1. Ethidium bromide concentration in the PCR reaction mixture was 5.0 μg/ml and primers amounts were from 0.5 to 100 pmole.

To verify if ethidium bromide was equally effective with different amounts of template, were added different amounts of mtDNA carrying cytosine nucleotide (C) at position 16311 to the reaction containing either primer 16311A (SEQ ID N° 3) or 16311G (SEQ ID N° 4).

The PCR was carried out as in the example 1. Ethidium bromide concentration in the PCR reaction mixture was 5.0 μg/ml, template amount was from 0.5 ng to 500 ng.

Example 4: Adding ethidium bromide at different time

In order to asses if it was necessary to add the intercalating agent before the primer and the template got together in the reaction, we performed PCR amplifications adding the ethidium bromide before, in between and after the addition of the primer and the template.

The PCR was carried out as in the example 1 adding 5.0 μg/ml of ethidium bromide before, between and after the primers and template.

Example 5: PCR amplification using proofreading enzyme instead Taq DNA Polymerase

PCR reaction mixture and conditions:

Proofreading amplifications were performed with 0.6 U of *Pfu* DNA Polymerase (Promega Corp.) in 20 mM Tris-HCl (pH 8.8), 10mM KCl, 0.1% Triton® X-100, 2 mM MgSO4, 10nM (NH4)SO4, 0.1 mg/ml BSA, 200uM each deoxy-NTP, 25 pmol of upstream primer, 10 pmol of downstream primer and 10 ng of total DNA under the same cycling conditions described in the example 1, except of increasing concentrations of ethidium bromide (from 4.5 to 5.5 μg/ml) were added. PCR products were electrophoresed through a 2% agarose gel and visualised with ethidium bromide.

Primers and templates:

The 299 bp region of the mtDNA (16,031-16,330) carrying cytosine nucleotide (C) at position 16311 was amplified with Pfu DNA Polymerase (Promega) in four separate PCR reactions using primer FW I (sequence showed above) and the four alternative downstream primers 16311G (SEQ ID N° 4), 16311A (SEQ ID N° 3), 16311C (SEQ ID N° 6) and 16311T (SEQ ID N° 5) (sequences showed above).

Example 6: Detection of a single-base mutation in exon 8 of *Homo sapiens* Cytochrome b-245 beta polypeptide (CYBB) gene using the method of the invention.

The PCR was carried out as in the example 1, adding 10 pmole of both primers and 5.0 μg/ml of ethidium bromide. The sequence of the primers was:

CYBB8FW Upstream primer 5'-CTC CCT CTG AAT ATT TTG TTA TC-3' (SEQ ID N° 15)
CYBB8A Downstream primer 5'-GAC CAC CTT CTG TTG AGA TCA-3' (SEQ ID N° 16)
CYBB8G Downstream primer 5'-GAC CAC CTT CTG TTG AGA TCG-3' (SEQ ID N° 17)
CYBB8T Downstream primer 5'-GAC CAC CTT CTG TTG AGA TCT-3' (SEQ ID N° 18)
CYBB8C Downstream primer 5'-GAC CAC CTT CTG TTG AGA TCC-3' (SEQ ID N° 19)

Example 7: Fidelity of *Taq* DNA Polymerase is enhanced by ethidium bromide

It has been previously demonstrated in the present application than the intercalating agent ethidium bromide helps to prevent the incorporation of nucleotides in the presence of a primer carrying a mismatch at the 3'end. If this enhanced primer selectivity occurred in every single nucleotide addition during the polymerization reaction it would then render a more accurate final product, regardless of the existence of any editing function. In order to demonstrate this the LacI test was performed as follow:

Overview of the LacI test:

A 2.5 kb fragment encoding *orilac$^q$IOZ'* was PCR amplified with primers containing 5' *Nsi*I and *Nde*I restriction sites and digested with these two restriction enzymes. It was then ligated into a previously *Nsi*I/*Nde*I-digested 1.3 kb fragment that contains the β-lactamase sequence. The resulting plasmid was transformed into *E.coli* JM109 competent cells and plated on agar with ampicilin (100μg/ml) plus X-gal (80μg/ml). Blue and white colonies were counted.

Construction of the p3ZIQ24 template:

The truncated *lac*I gene (SEQ ID N° 20) of the pGEM®-3Z vector (Promega Corp.- SEQ ID N° 21) was substituted by a functionral copy of the *lac*I$^q$ repressor gene derived from the *E.coli* strain HB101. The 1,389 bp *lac*I$^q$ fragment was obtained by amplifying the *lac*I region of HB101 with primers lacIFw (SEQ ID N° 23) and lacIRv (SEQ ID N° 22) . The latter carries a C to T transition at the position -35 of the gene, conferring the strong *lac*I$^q$ promoter genotype to the construct (Calos, P.M. (1978) *Nature* 274:762). The *lac*I$^q$ fragment was cloned blunt on a Pst I-digested pGem 3Z plasmid lacking the 379bp *Pst* I-*Pst* I region. The new plasmid of 3,753 bp (FIG: 10-a), named p3ZIQ24, was used as the template for the PCR fidelity assays.

Polymerase chain reactions:

PCR amplifications (Mullis, K.B. (1990) *Ann. Biol. Clin.* 48:579) were performed in a MJ Research PTC-150 thermal cycler in a 25 μl reaction volume containing 10 mM Tris-HCl (pH 9.0), 50mM KCl, 0.1% Triton® X-100, 1.5 mM MgCl2, 200uM each deoxy-NTP, 1.2 U *Taq* DNA Polymerase (Promega Corp.) or *Tli* DNA Poymerase(Promega Corp.), 100 pmole of each primer and 0.1 ng of p3ZIQ24 as template. Cycling conditions included an initial denaturalisation step of 4 min at 94°C, followed by 36 cycles of 30 sec. at 94°C, 30 sec. at 50°C and 4 min. at 72°C . PCR products were electrophoresed through a 0.7% TAE-agarose gel and visualised under UV with ethidium bromide. The input and output DNA was quantified by gel densitometry with the AlphaEase® software (Alphalnnotech Inc.).

Cloning of the PCR products

A 2482 bp *lacI*$^q$ fragment carrying the origin region of p3ZIq24 was generated by PCR with primers Orifw (SEQ ID N° 27) and OriRv (SEQ ID N° 26). A 1270 bp fragment having the beta-lactamase region of p3ZIq24 was obtained with primers AmpRv (SEQ ID N° 24) and Ampfw (SEQ ID N° 25). Since primers OriRv and Ampfw carry a *Nsi*I restriction site and primers Orifw and AmpRv a *Nde*I site both fragments were digested with the restriction enzymes *Nsi*I and *Nde*I to create cohesive ends. They were then purified with Wizard® SV Gel and PCR Clean Up (Promega) and ligated in a 1:1 ratio overnight at 18°C. The resulting plasmids were transformed in triplicates in *E.coli* JM109 competent cells (Sambrook, J. Fritsch, E.F. and Maniatis, T. (1989). *Molecular Cloning A Laboratory Manual.* Second edition. Cold Spring Harbor Laboratory Press 1.74) and plated on LB-Amp X-Gal plates.

LacI based Assay:

After overnight incubation at 37°C blue and white colonies were counted. The error rate (f) was calculated according to the following formula (Keohavong, P. and Thiliy, W.G. (1989) *Proc.Natl.Acad.Sci.* 86:9253):

$$f = -1nF/ d \times b$$

where F is the fraction of white colonies over the total, d is the number of duplications (2d=output DNA/input DNA) and b is the effective target site, which is 349 bp for lacI (Provost, G.S., Kretz, P.L., Hamner, R.T., Matthews, C.D., Rogers, B.J., Lundberg, K.S., Dycaico, M.J. and Short, J.M. (1993) *Mut.Research* 288:133).

Results:

Table 1 illustrates that the ethidium bromide treatment improves the fidelity performance of *Taq* DNA polymerase. Using a 2µg/ml concentration of ethidium bromide the error rate is 1.3 x $10^{-5}$, comparing to 2.1 x$10^{-5}$ without treatment. The error rate determined for *Taq* DNA polymerase agrees with the figures published in the literature (Lundberg, K.S., Shoemaker, D.D., Adams, M.W., Short, J.M., Sorge, J.A. and Mathur, E.J. (1991) *Gene* 108:1). The error rate with ethidium bromide was reduced by almost a half. Figure 10-b shows that the yield of the 2, 482 bp PCR product is somewhat lower in the presence of the intercalating agent, a feature common to many high fidelity reactions. According to this and as it was mentioned previously in this present application nucleic acid sequences may be accurate amplified without the use of any other reagent than a correct amount of ethidium bromide.

Table 1

| | P3ZIQ24 (ng) | Produc t (ng) | d | N° Blue Colonies | N° White Colonies | N° Total Colonies | % *lacI*- | F |
|---|---|---|---|---|---|---|---|---|
| EtBr 0µg/ ml | 0.1 | 3000 | 14.9 | 65 | 440 | 505 | 12.87 | |
| EtBr 0µg/ml | 0.1 | 3000 | 14.9 | 52 | 499 | 551 | 9.44 | |
| EtBr 0µg/ ml | 0.1 | 3000 | 14.9 | 47 | 447 | 494 | 9.51 | |
| **TOTAL** | | | | 164 | 1386 | 1550 | 10.58 | 2.1x$10^{-5}$ |
| EtBr 2µg/ ml | 0.1 | 50 | 9 | 23 | 630 | 653 | 3.52 | |
| EtBr 2µg/ ml | 0.1 | 50 | 9 | 32 | 748 | 780 | 4.10 | |
| EtBr 2µg/ ml | 0.1 | 50 | 9 | 26 | 520 | 546 | 4.76 | |
| **TOTAL** | | | | 81 | 1898 | 1979 | 4.09 | 1.3x$10^{-5}$ |

While preferred embodiments of the present invention have been illustrated and described, it will be obvious to those skilled in the art that various changes and modifications may be made therein without departing from the scope of the invention as defined in the appended claims.

## Claims

1. A method for increasing the selectivity extension by polymerase enzymes when the 3' end of a primer does not hybridize perfectly with the target, wherein said primer is an added primer or a growing nucleic acid chain that is being polymerized, the growing nucleic acid chain acting as a primer for the addition of the next nucleotide of the sequence, the method comprising the steps of:

   (a) obtaining a nucleic acid sample;
   (b) hybridizing said nucleic acid sample to said primer;
   (c) subjecting said nucleic acid sample hybridized to a extension reaction by extending the primer with a polymerizing enzyme, wherein the reaction extension mixture medium contains an intercalating agent; and
   (d) detecting the presence of extension products.

2. The method of claim 1, wherein the extension reaction by extending a primer with a polymerizing enzyme in step (c) is the extension reaction step in a PCR procedure and the extension reaction medium is a PCR reaction mixture, wherein said PCR reaction mixture contains the intercalating agent and, the step (d) is the detection of amplified products.

3. The method of claim 1, wherein the intercalating agent is a flat organic molecule capable of stacking between the nucleic acid bases.

4. The method of claim 3, wherein the intercalating agent is selected from the group consisting of ethidium bromide, dihydroethidium, ethidium homodimer-1, ethidium homodimer-2, acridine, propidium iodide, YOYO®-1 and TOTO®-1.

5. The method of claim 3, wherein the intercalating agent is ethidium bromide.

6. The method of claim 2, wherein the intercalating agent concentration is about 1 to 7 µg/ml in the PCR reaction mixture.

7. The method of claim 2, wherein one or more primers is/are employed in the PCR.

8. The method of claim 1, wherein the polymerizing enzyme is selected from the group consisting of and DNA or RNA polymerases, with or without editing activity, native enzyme or recombinant enzyme, thermostable enzyme or non-thermostable enzyme, complete enzyme or some active fragment.

9. A method for reducing primer extension by polymerase enzymes when the 3' end of a primer does not hybridize perfectly with the target, to increase the selectivity of single nucleotide mutation or gene analyses, whereby the false positive results are suppressed, the method comprising the steps of:

   (a) obtaining a nucleic acid sample;
   (b) hybridizing said nucleic acid sample to a primer;
   (c) subjecting said nucleic acid sample hybridized to a extension reaction by extending the primer with a polymerizing enzyme, wherein the reaction extension mixture medium contains an intercalating agent; and
   (d) detecting the presence of extension products.

10. The method of claim 9, wherein the step (b) comprises hybridized the nucleic acid sample to primers in a monoplex or multiplex format containing either one or more sets of allelic primers.

11. The method of claim 10, wherein de presence of ethidium bromide in the extension reaction (i) decreases the amount of extension products when the 3' end of a primer does not hybridize perfectly with the target sequence, and/or (ii) increases the selectivity detection of single nucleotide mutation and/or (iii) suppresses false positive extension products in gene analyses.

12. The method of claim 9, wherein the extension reaction by extending a primer with a polymerizing enzyme in step (c) is the extension reaction steps in a PCR procedure and the extension reaction medium is a PCR reaction mixture, wherein said PCR reaction mixture contains an intercalating agent and, the step (d) is the detection of amplified products.

13. The method of claim 9, wherein the intercalating agent is a flat organic molecule capable of stacking between the nucleic acid bases.

14. The method of claim 13, wherein the intercalating agent is selected from the group consisting of ethidium bromide, dihydroethidium, ethidium homodimer-1, ethidium homodimer-2, acridine, propidium iodide, YOYO®-1 and TOTO®-1.

15. The method of claim 13, wherein the intercalating agent is ethidium bromide.

16. The method of claim 13, wherein the intercalating agent concentration is about 1 to 7 μg/ml in the PCR reaction mixture.

17. The method of claim 12, wherein one or more primers is/are employed in the PCR.

18. The method of claim 12, wherein the polymerizing enzyme is selected from the group consisting of DNA or RNA polymerases, with or without editing activity, native enzyme or recombinant enzyme, thermostable enzyme or non-thermostable enzime, complete enzyme or some active fragment.

19. A method for increasing the fidelity of polymerase reactions comprising the steps of:

   (a) obtaining a nucleic acid sample;
   (b) hybridizing said nucleic acid sample to a primer;
   (c) subjecting said nucleic acid sample hybridized to a extension reaction by extending the primer with a polymerizing enzyme, wherein the reaction extension mixture medium contains an intercalating agent; and
   (d) detecting the presence of extension products.

20. The method of claim 19, wherein the extension reaction by extending a primer with a polymerizing enzyme in step (c) is the extension reaction steps in a PCR procedure and the extension reaction medium is a PCR reaction mixture, wherein said PCR reaction mixture contains an intercalating agent and, the step (d) is the detection of amplified products.

**21.** The method of claim 20, wherein the intercalating agent is a flat organic molecule capable of stacking between the nucleic acid bases.

**22.** The method of claim 21, wherein the intercalating agent is selected from the group consisting of ethidium bromide, dihydroethidium, ethidium homodimer-1, ethidium homodimer-2, acridine, propidium iodide, YOYO®-1 and TOTO®-1.

**23.** The method of claim 21, wherein the intercalating agent is ethidium bromide.

**24.** The method of claim 20, wherein the intercalating agent concentration is about 1 to 7 µg/ml in the PCR reaction mixture.

**25.** The method of claim 20, wherein one or more primers is/are employed in the PCR.

**26.** The method of claim 20, wherein the polymerizing enzyme is selected from the group consisting of DNA or RNA polymerases, with or without editing activity, native or recombinant, thermostable or non-thermostable, complete enzyme or some active fragment.

**27.** A mixture reagent for an extension reaction, wherein the mixture reagent comprises:

a polymerizing enzyme, dNTPs, a buffer, an intercalating agent, primers, template and salts.

**28.** The mixture reagent of claim 27, wherein the intercalating agent is a flat organic molecule capable of stacking between the nucleic acid bases.

**29.** The mixture reagent of claim 28, wherein the intercalating agent is selected from the group consisting of ethidium bromide, dihydroethidium, ethidium homodimer-1, ethidium homodimer-2, acridine, propidium iodide, YOYO®-1 and TOTO®-1 .

**30.** The mixture reagent of claim 28, wherein the intercalating agent is ethidium bromide and the concentration of ethidium bromide is about 1 to 7µg/ml.

**31.** The mixture reagent of claim 27, wherein said mixture reagent is a PCR reaction mixture.

**32.** The mixture reagent of claim 27, wherein the polymerizing enzyme is selected from the group consisting of DNA or RNA polymerases, with or without editing activity, native or recombinant, thermostable or non-thermostable, complete enzyme or some active fragment

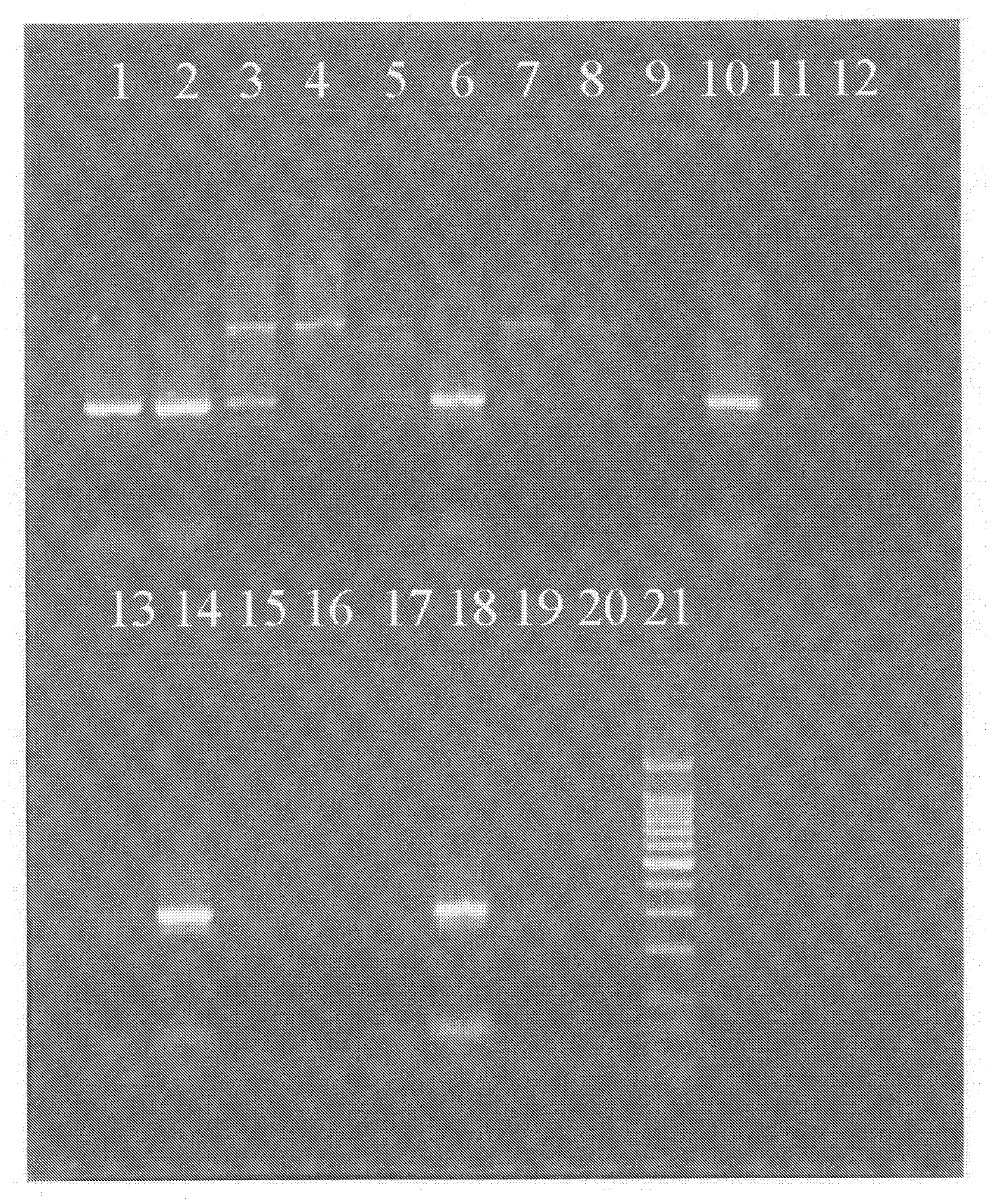


FIG. 1

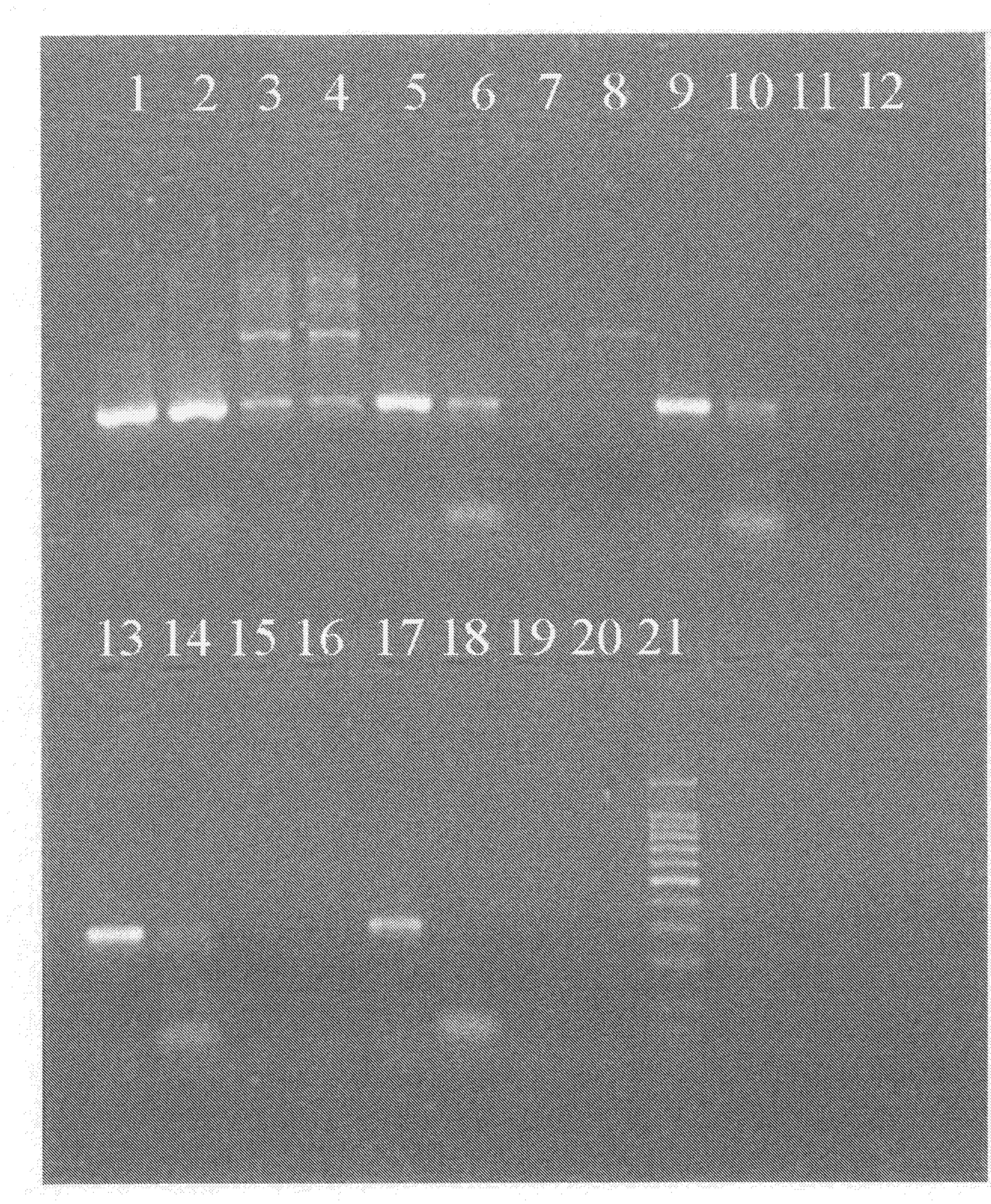


FIG. 2

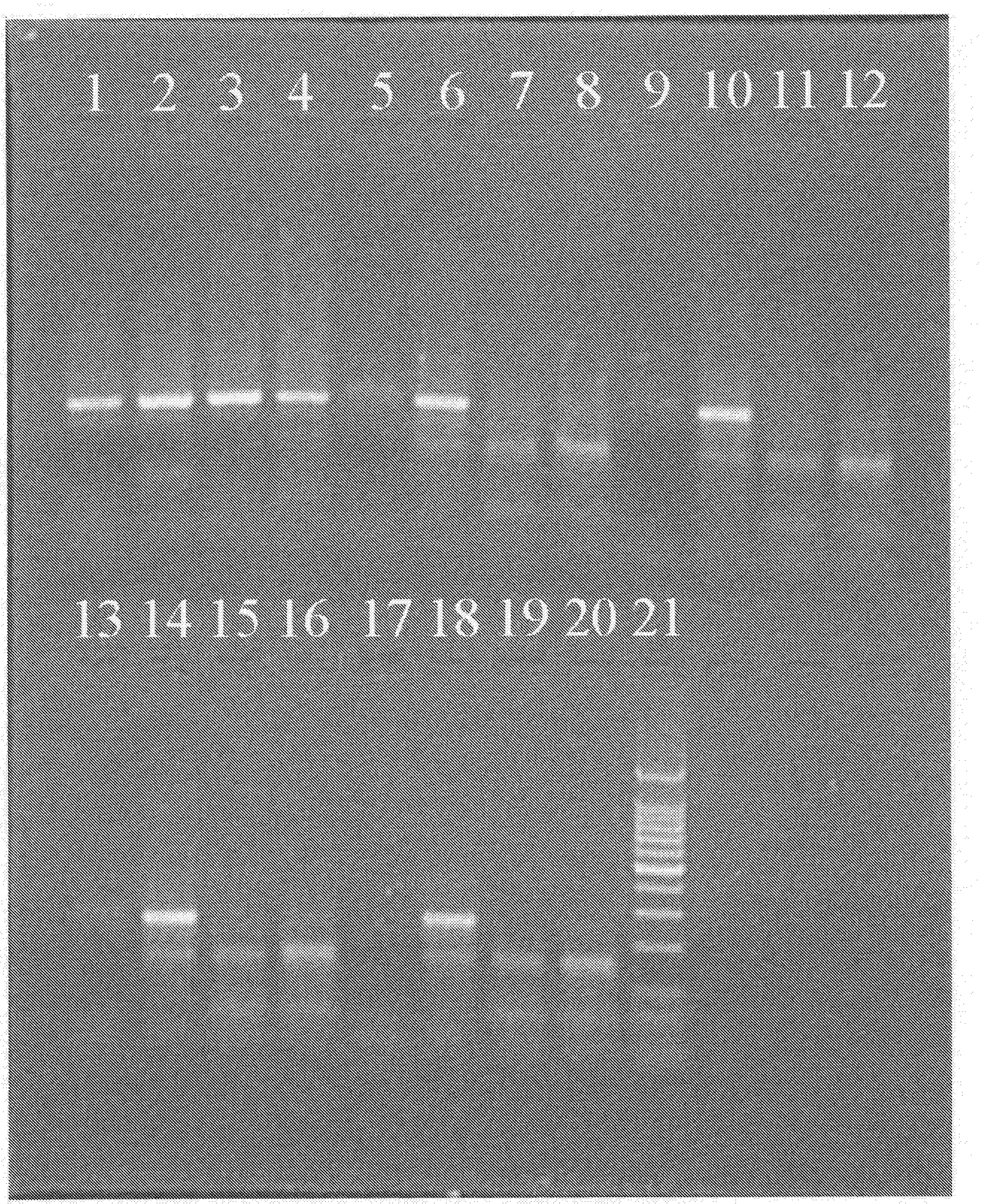


FIG. 3

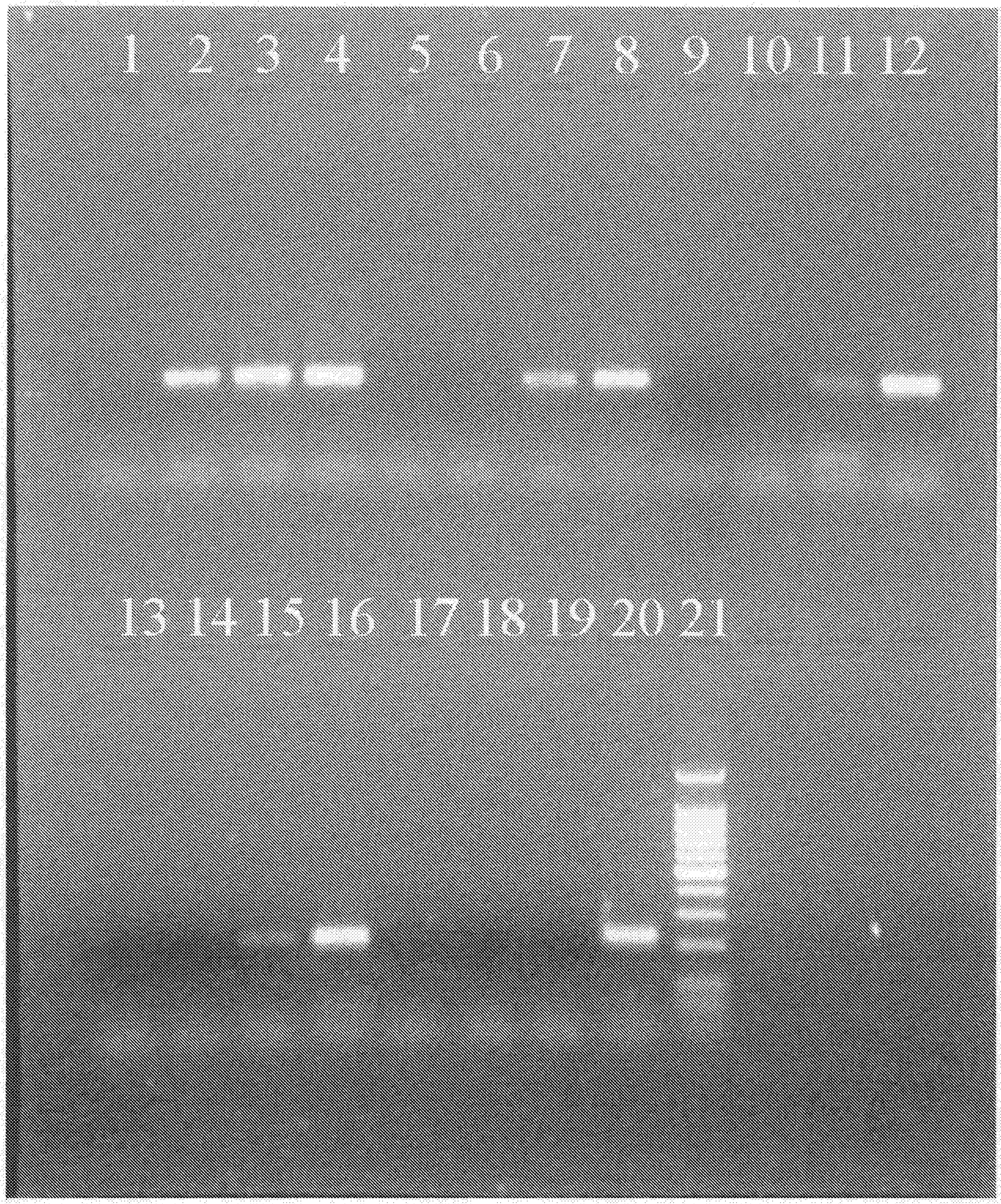


FIG. 4

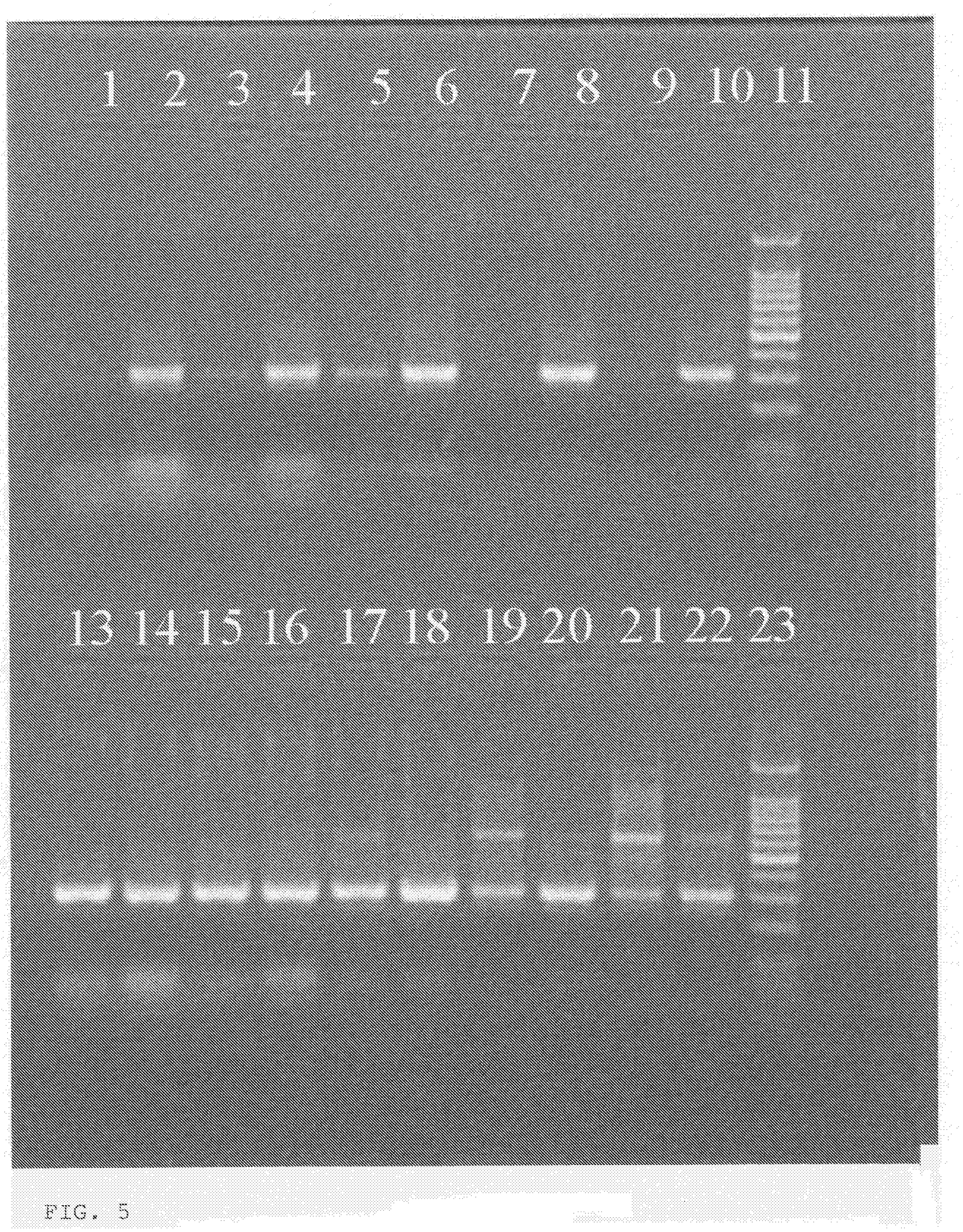


FIG. 5

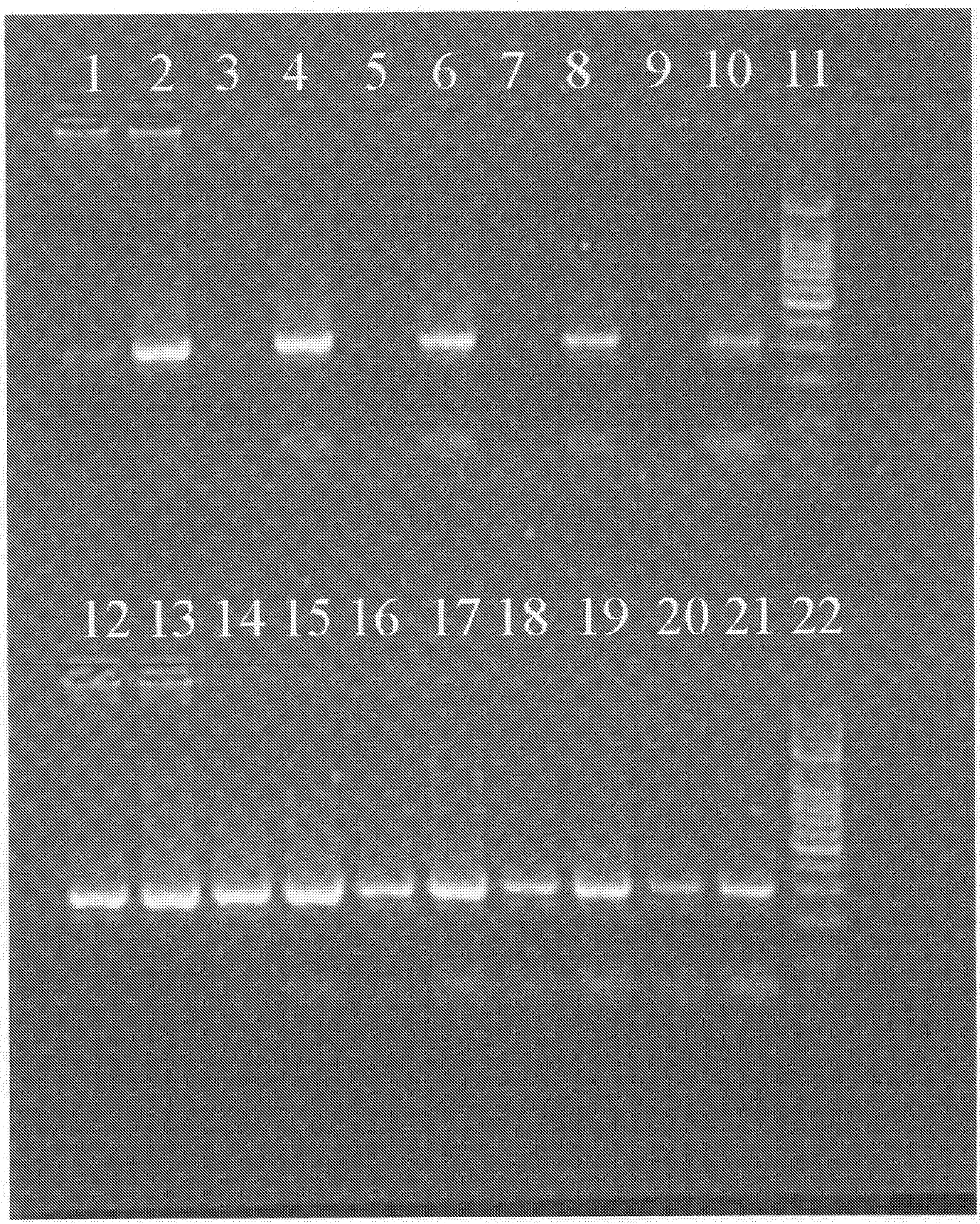


FIG. 6

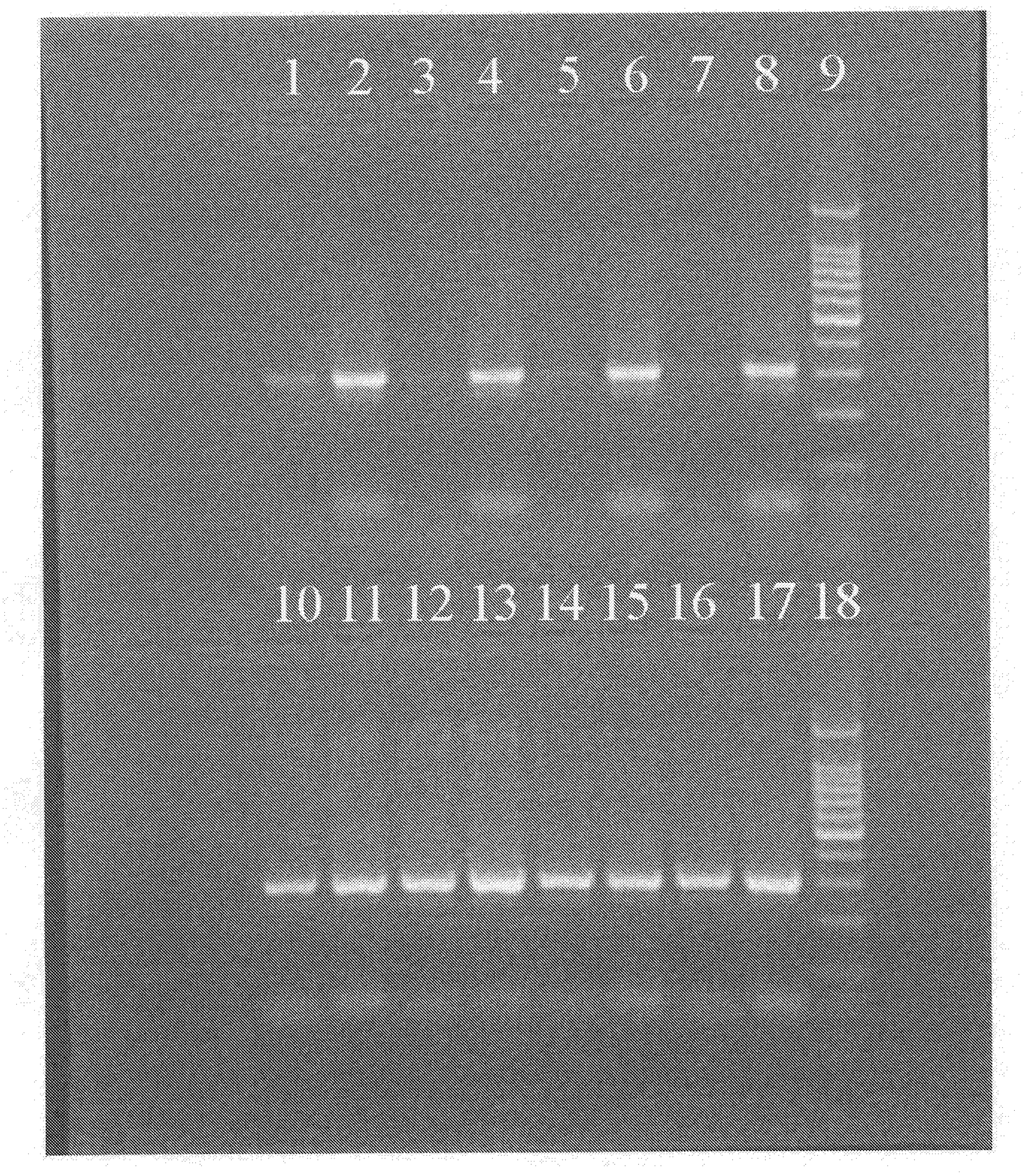


FIG. 7

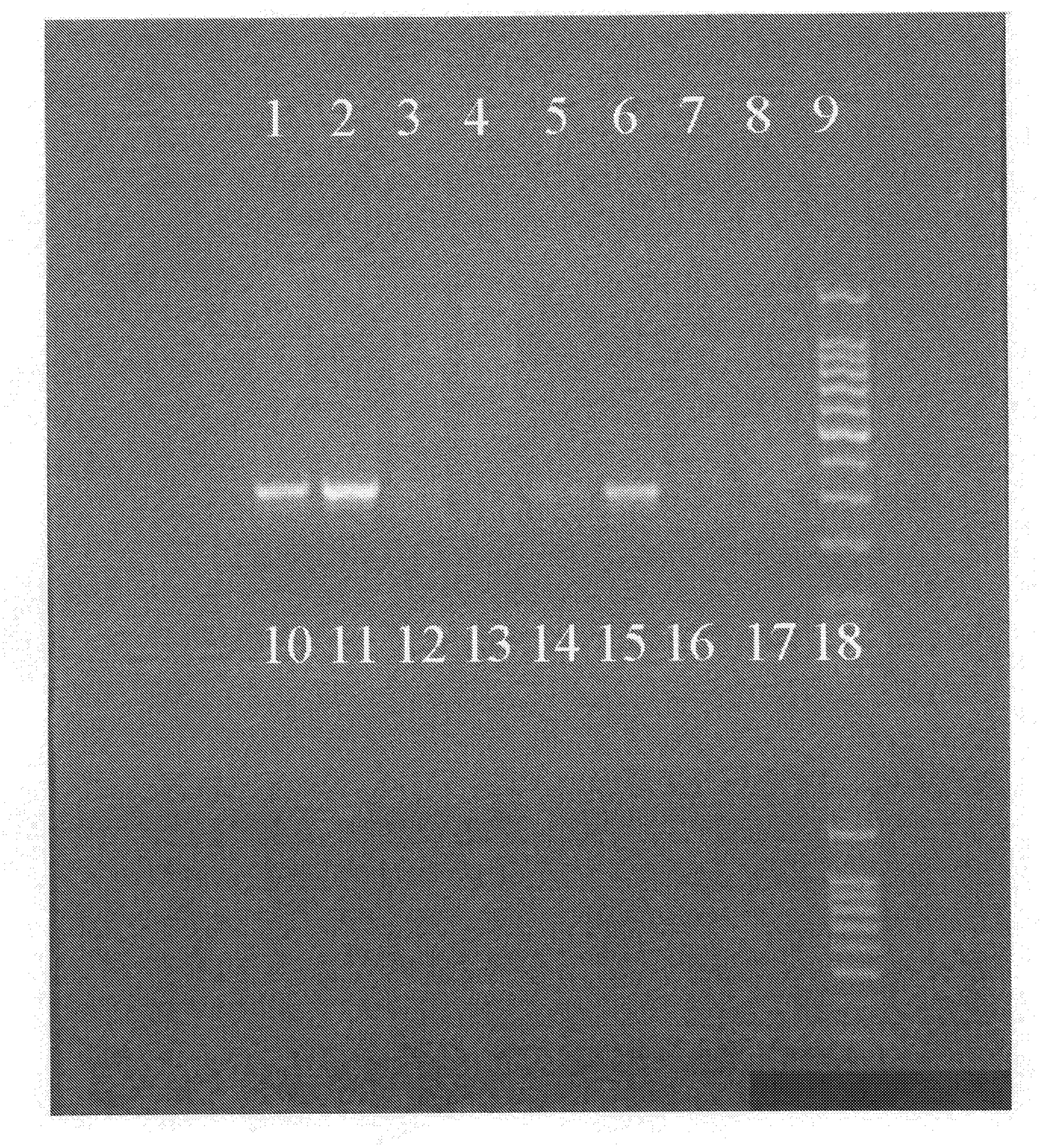


FIG. 8

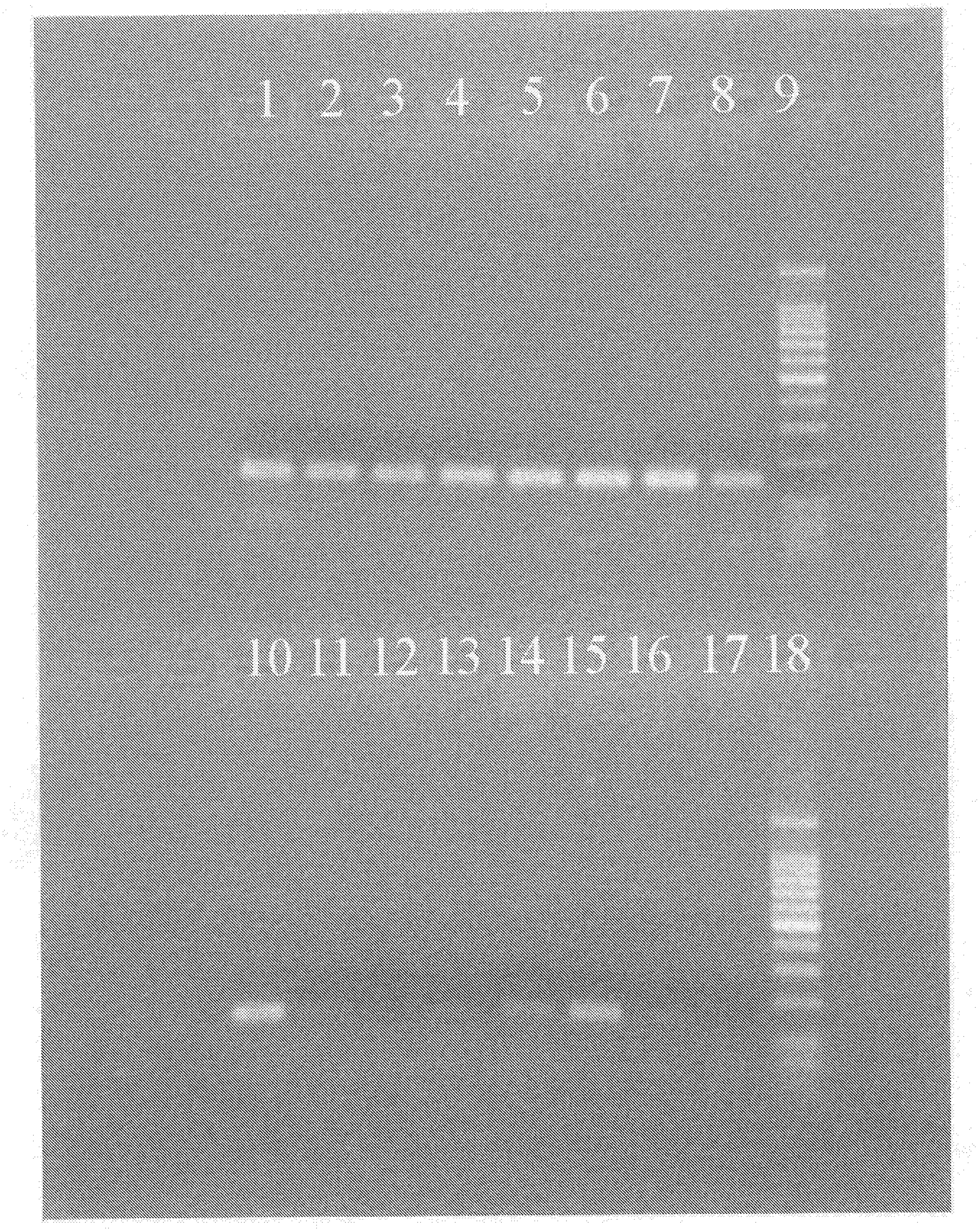


FIG. 9

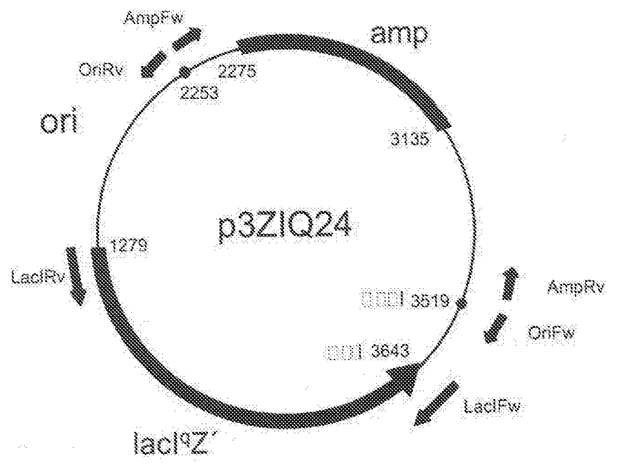


FIG. 10A

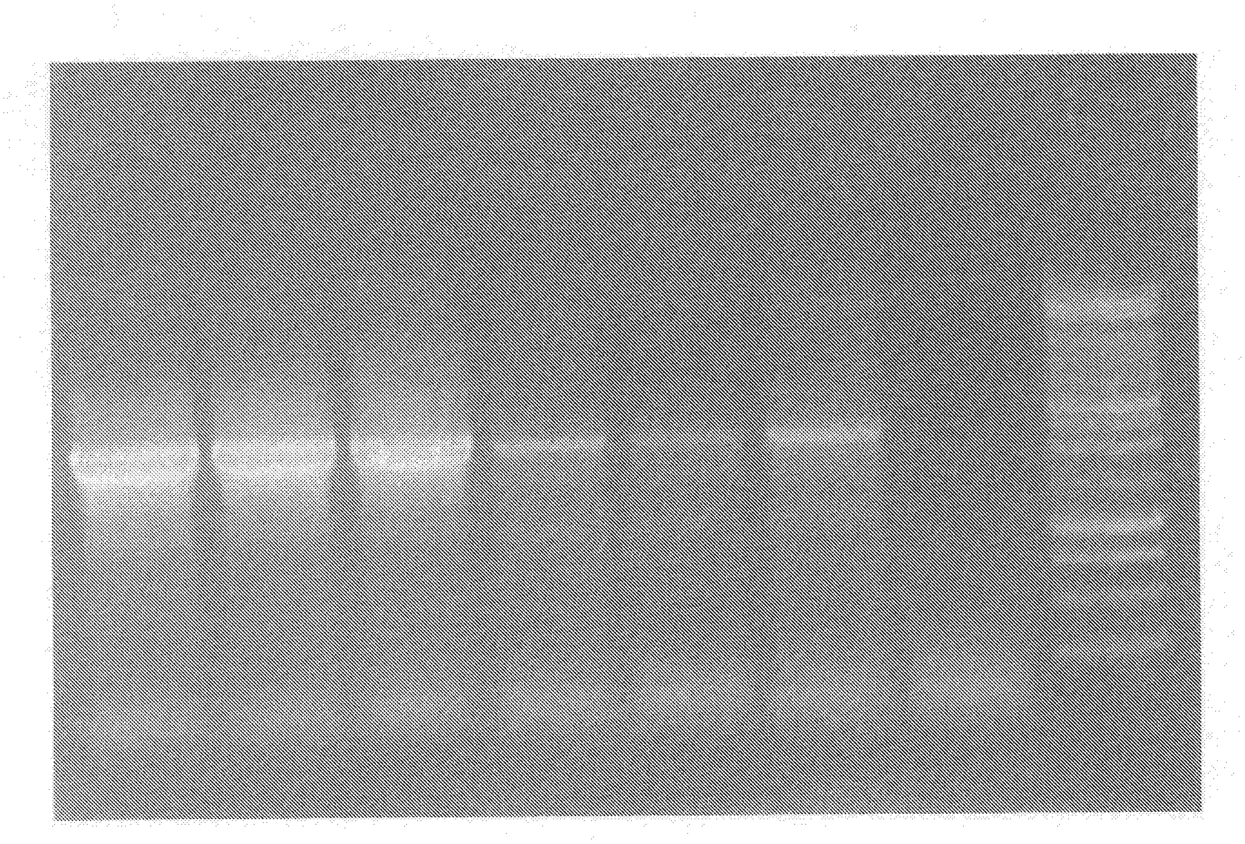

FIG. 10B

European Patent Office

# EUROPEAN SEARCH REPORT

Application Number
EP 04 10 2641

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X<br><br>Y | EP 1 256 631 A (PE CORP NY) 13 November 2002 (2002-11-13)<br>* page 12, line 54 - page 13, line 10; example 5 * | 27-32<br><br>1-26 | C12Q1/68 |
| X<br><br>A | US 2002/058258 A1 (RASMUSSEN RANDY P ET AL) 16 May 2002 (2002-05-16)<br>* page 13, column 1, paragraph 245 - column 2, paragraph 246 * | 27-32<br><br>1-26 | |
| X<br><br>A | US 6 171 785 B1 (HIGUCHI RUSSELL G) 9 January 2001 (2001-01-09)<br>* column 12, line 64 - column 13, line 9 *<br>* column 14, line 18 - line 37 *<br>* examples 1,2 * | 27-32<br><br>1-26 | |
| Y | RUSSOM AMAN ET AL: "Single nucleotide polymorphism analysis by allele-specific primer extension with real-time bioluminescence detection in a microfluidic device." JOURNAL OF CHROMATOGRAPHY. A. 3 OCT 2003, vol. 1014, no. 1-2, 3 October 2003 (2003-10-03), pages 37-45, XP002297120<br>* the whole document * | 1-18 | TECHNICAL FIELDS SEARCHED (Int.Cl.7)<br>C12Q |
| Y | US 5 976 842 A (WURST HELMUT) 2 November 1999 (1999-11-02)<br>* the whole document * | 1-8, 19-26 | |
| A | EP 1 026 261 A (ORTHO CLINICAL DIAGNOSTICS INC) 9 August 2000 (2000-08-09)<br>* the whole document * | 1-26 | |
| | -/-- | | |

The present search report has been drawn up for all claims

| Place of search | Date of completion of the search | Examiner |
|---|---|---|
| The Hague | 28 September 2004 | Schmitt, A |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 04 10 2641

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | BAILLY C ET AL: "Drug-DNA sequence-dependent interactions analysed by electric linear dichroism." JOURNAL OF MOLECULAR RECOGNITION : JMR. DEC 1992, vol. 5, no. 4, December 1992 (1992-12), pages 155-171, XP008035695 ISSN: 0952-3499 * figure 1; table 1 * ----- | | |
| | | | TECHNICAL FIELDS SEARCHED (Int.Cl.7) |

The present search report has been drawn up for all claims

| Place of search | Date of completion of the search | Examiner |
|---|---|---|
| The Hague | 28 September 2004 | Schmitt, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

3

EPO FORM 1503 03.82 (P04C01)

European Patent Office

Application Number

EP 04 10 2641

## CLAIMS INCURRING FEES

The present European patent application comprised at the time of filing more than ten claims.

- [ ] Only part of the claims have been paid within the prescribed time limit. The present European search report has been drawn up for the first ten claims and for those claims for which claims fees have been paid, namely claim(s):

- [ ] No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [X] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [ ] None of the further search fees have been paid within the fixed time limit. The present European search report has been drawn up for those parts of the European patent application which relate to the invention first mentioned in the claims, namely claims:

European Patent Office

## LACK OF UNITY OF INVENTION SHEET B

Application Number

EP 04 10 2641

The Search Division considers that the present European patent application does not comply with the requirements of unity of invention and relates to several inventions or groups of inventions, namely:

1. claims: 9-18 (completely), claims 1-8, 27-32 (partially)

   Method and composition for reducing primer extension by polymerase enzymes when the 3'-end of a primer does not hybridize perfectly with the target.

   ---

2. claims: 19-26 (completely), claims 1-8, 27-32 (partially)

   Method and composition for increasing the fidelity of polymerase reactions.

   ---

## ANNEX TO THE EUROPEAN SEARCH REPORT ON EUROPEAN PATENT APPLICATION NO. EP 04 10 2641

This annex lists the patent family members relating to the patent documents cited in the above-mentioned European search report.
The members are as contained in the European Patent Office EDP file on
The European Patent Office is in no way liable for these particulars which are merely given for the purpose of information.

28-09-2004

| Patent document cited in search report | Publication date | Patent family member(s) | Publication date |
|---|---|---|---|
| EP 1256631 A | 13-11-2002 | US 5994056 A | 30-11-1999 |
| | | EP 1256631 A1 | 13-11-2002 |
| | | EP 0872562 A1 | 21-10-1998 |
| | | AT 184322 T | 15-09-1999 |
| | | AT 223970 T | 15-09-2002 |
| | | AU 665185 B2 | 21-12-1995 |
| | | AU 1513892 A | 05-11-1992 |
| | | BR 9201618 A | 15-12-1992 |
| | | CA 2067909 A1 | 03-11-1992 |
| | | CA 2218818 A1 | 03-11-1992 |
| | | DE 1256631 T1 | 27-11-2003 |
| | | DE 69229929 D1 | 14-10-1999 |
| | | DE 69229929 T2 | 18-05-2000 |
| | | DE 69232773 D1 | 17-10-2002 |
| | | DE 69232773 T2 | 07-08-2003 |
| | | DK 512334 T3 | 03-04-2000 |
| | | DK 872562 T3 | 30-12-2002 |
| | | EP 0512334 A2 | 11-11-1992 |
| | | ES 2137164 T3 | 16-12-1999 |
| | | ES 2183256 T3 | 16-03-2003 |
| | | JP 3136129 B2 | 19-02-2001 |
| | | JP 10201464 A | 04-08-1998 |
| | | JP 3007477 B2 | 07-02-2000 |
| | | JP 5184397 A | 27-07-1993 |
| | | NO 921731 A | 03-11-1992 |
| | | NZ 242565 A | 26-07-1994 |
| | | US 6171785 B1 | 09-01-2001 |
| | | ZA 9202990 A | 27-01-1993 |
| US 2002058258 A1 | 16-05-2002 | US 6232079 B1 | 15-05-2001 |
| | | US 6174670 B1 | 16-01-2001 |
| | | US 2004002098 A1 | 01-01-2004 |
| | | US 6245514 B1 | 12-06-2001 |
| | | AU 726501 B2 | 09-11-2000 |
| | | AU 3481297 A | 05-01-1998 |
| | | CA 2257109 A1 | 11-12-1997 |
| | | EP 1179600 A1 | 13-02-2002 |
| | | EP 0912766 A1 | 06-05-1999 |
| | | EP 1033411 A2 | 06-09-2000 |
| | | JP 2000509608 T | 02-08-2000 |
| | | NZ 333136 A | 27-03-2000 |
| | | NZ 502323 A | 28-09-2001 |
| | | WO 9746714 A1 | 11-12-1997 |
| | | AT 260988 T | 15-03-2004 |
| | | AU 727296 B2 | 07-12-2000 |
| | | AU 3154797 A | 05-01-1998 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 04 10 2641

This annex lists the patent family members relating to the patent documents cited in the above-mentioned European search report.
The members are as contained in the European Patent Office EDP file on
The European Patent Office is in no way liable for these particulars which are merely given for the purpose of information.

28-09-2004

| Patent document cited in search report | Publication date | Patent family member(s) | Publication date |
|---|---|---|---|
| US 2002058258 A1 | | AU 729644 B2 | 08-02-2001 |
| | | AU 3380097 A | 05-01-1998 |
| | | CA 2256612 A1 | 11-12-1997 |
| | | CA 2256773 A1 | 11-12-1997 |
| | | DE 69727932 D1 | 08-04-2004 |
| | | EP 1442794 A2 | 04-08-2004 |
| | | EP 0912760 A2 | 06-05-1999 |
| | | EP 0906449 A2 | 07-04-1999 |
| | | JP 2000512138 T | 19-09-2000 |
| | | JP 2000511435 T | 05-09-2000 |
| | | KR 2000016161 A | 25-03-2000 |
| | | KR 2000016326 A | 25-03-2000 |
| | | NZ 333135 A | 28-02-2000 |
| | | NZ 333137 A | 27-03-2000 |
| | | WO 9746707 A2 | 11-12-1997 |
| | | WO 9746712 A2 | 11-12-1997 |
| US 6171785 B1 | 09-01-2001 | US 5994056 A | 30-11-1999 |
| | | AT 184322 T | 15-09-1999 |
| | | AT 223970 T | 15-09-2002 |
| | | AU 665185 B2 | 21-12-1995 |
| | | AU 1513892 A | 05-11-1992 |
| | | BR 9201618 A | 15-12-1992 |
| | | CA 2067909 A1 | 03-11-1992 |
| | | CA 2218818 A1 | 03-11-1992 |
| | | DE 1256631 T1 | 27-11-2003 |
| | | DE 69229929 D1 | 14-10-1999 |
| | | DE 69229929 T2 | 18-05-2000 |
| | | DE 69232773 D1 | 17-10-2002 |
| | | DE 69232773 T2 | 07-08-2003 |
| | | DK 512334 T3 | 03-04-2000 |
| | | DK 872562 T3 | 30-12-2002 |
| | | EP 1256631 A1 | 13-11-2002 |
| | | EP 0512334 A2 | 11-11-1992 |
| | | EP 0872562 A1 | 21-10-1998 |
| | | ES 2137164 T3 | 16-12-1999 |
| | | ES 2183256 T3 | 16-03-2003 |
| | | JP 3136129 B2 | 19-02-2001 |
| | | JP 10201464 A | 04-08-1998 |
| | | JP 3007477 B2 | 07-02-2000 |
| | | JP 5184397 A | 27-07-1993 |
| | | NO 921731 A | 03-11-1992 |
| | | NZ 242565 A | 26-07-1994 |
| | | ZA 9202990 A | 27-01-1993 |
| US 5976842 A | 02-11-1999 | NONE | |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 04 10 2641

This annex lists the patent family members relating to the patent documents cited in the above-mentioned European search report.
The members are as contained in the European Patent Office EDP file on
The European Patent Office is in no way liable for these particulars which are merely given for the purpose of information.

28-09-2004

| Patent document cited in search report | Publication date | Patent family member(s) | Publication date |
|---|---|---|---|
| EP 1026261 A | 09-08-2000 | AU 764914 B2 | 04-09-2003 |
| | | AU 1487700 A | 10-08-2000 |
| | | CA 2295942 A1 | 03-08-2000 |
| | | CN 1271019 A | 25-10-2000 |
| | | EP 1026261 A2 | 09-08-2000 |
| | | JP 2000279184 A | 10-10-2000 |
| | | NO 20000538 A | 04-08-2000 |
| | | US 6300075 B1 | 09-10-2001 |

EPO FORM P0459

For more details about this annex : see Official Journal of the European Patent Office, No. 12/82